(19) Europäisches Patentamt / European Patent Office / Office européen des brevets

(11) **EP 3 901 278 B1**

(12) **EUROPEAN PATENT SPECIFICATION**

(45) Date of publication and mention of the grant of the patent:
**23.10.2024 Bulletin 2024/43**

(21) Application number: **21150302.4**

(22) Date of filing: **19.05.2015**

(51) International Patent Classification (IPC):
***C12Q 1/686*** (2018.01)  ***C12Q 1/6858*** (2018.01)

(52) Cooperative Patent Classification (CPC):
(C-Sets available)
**C12Q 1/686; C12Q 1/6858**  (Cont.)

(54) **ALLELE-SPECIFIC AMPLIFICATION USING A COMPOSITION OF OVERLAPPING NON-ALLELE-SPECIFIC PRIMER AND ALLELE-SPECIFIC BLOCKER OLIGONUCLEOTIDES**

ALLELSPEZIFISCHE AMPLIFIZIERUNG MIT EINER ZUSAMMENSETZUNG AUS ÜBERLAPPENDEM NICHT-ALLELSPEZIFISCHEM PRIMER UND ALLELSPEZIFISCHEN BLOCKEROLIGONUKLEOTIDEN

AMPLIFICATION SPÉCIFIQUE D'UN ALLÈLE AU MOYEN D'UNE COMPOSITION D'AMORCE NON SPÉCIFIQUE D'ALLÈLE DE CHEVAUCHEMENT ET D'OLIGONUCLÉOTIDES BLOQUEURS SPÉCIFIQUES D'UN ALLÈLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2014 US 201462000114 P**

(43) Date of publication of application:
**27.10.2021 Bulletin 2021/43**

(62) Document number(s) of the earlier application(s) in accordance with Art. 76 EPC:
**15796383.6 / 3 146 080**

(73) Proprietor: **William Marsh Rice University**
**Houston, TX 77005 (US)**

(72) Inventors:
• **ZHANG, David Yu**
  **Houston, Texas 77030 (US)**
• **WU, Ruojia**
  **Houston, Texas 77054 (US)**
• **WANG, Juexiao**
  **Houston, Texas 77054 (US)**

(74) Representative: **Potter Clarkson**
**Chapel Quarter**
**Mount Street**
**Nottingham NG1 6HQ (GB)**

(56) References cited:
**WO-A1-2013/097173  WO-A1-2014/177540**

WO-A2-2011/146403  WO-A2-2012/151560
US-A1- 2010 009 355  US-A1- 2013 149 695
US-A1- 2014 017 685

• MURDOCK D G ET AL: "The age-related accumulation of a mitochondrial DNA control region mutation in muscle, but not brain, detected by a sensitive PNA-directed PCR clamping based method", NUCLEIC ACIDS RESEARCH, INFORMATION RETRIEVAL LTD, vol. 28, no. 21, 1 November 2000 (2000-11-01), pages 4350 - 4355, XP002310905, ISSN: 0305-1048
• WU LUCIA R ET AL: "Multiplexed enrichment of rare DNA variants via sequence-selective and temperature-robust amplification", NATURE BIOMEDICAL ENGINEERING, NATURE PUBLISHING GROUP UK, LONDON, vol. 1, no. 9, 4 September 2017 (2017-09-04), pages 714 - 723, XP036428896, DOI: 10.1038/S41551-017-0126-5

Remarks:
The complete document including Reference Table(s) and the Sequence Listing(s) can be downloaded from the EPO website

(52) Cooperative Patent Classification (CPC): (Cont.)

C-Sets
**C12Q 1/6858, C12Q 2525/161, C12Q 2527/107,
C12Q 2537/143, C12Q 2537/163;
C12Q 1/686, C12Q 2525/161, C12Q 2527/107,
C12Q 2537/143, C12Q 2537/163**

**Description**

**BACKGROUND**

Small differences in DNA and RNA sequence can lead to big differences in overall physical health and wellness of organisms including human beings. For example, a single-base change in a bacterial genome can lead to antibiotic resistance, and a single-base change in a human genome can lead to cancer progression. With the maturation of the genomics field and the accompanying discovery of many nucleic acid biomarker sequences and molecules, there is a strong demand from the biotechnology industry to develop reliable, robust, inexpensive, and precise nucleic acid assays that can discriminate single-base changes. In particular, many PCR-based assays have been developed that are allele-specific.

PCR-based approaches to the selective detection of rare mutations can be broadly classified into two families of approaches: (A) allele-specific primers, and (B) non-allele-specific primers with allele-specific blockers.

Examples of allele-specific primers include the amplification refractory mutation system (ARMS), allele-specific blocker PCR (ASB-PCR), and competitive allele specific TaqMan PCR (castPCR). Examples of (B) include PNA blocker PCR and co-amplification at lower denaturation temperature PCR (COLD-PCR).

US 2014/017685 entitled Methods, Compositions, and Kits for Determining the Presence/Absence of a Varian Nucleic Acid Sequence, was published on 16 January 2014, and described methods, compositions and kits for detecting the presence, absence or amount of a target nucleic acid or at least one variant nucleotide in one or more nucleic acids contained in a sample.

Prior to the present invention, allele-specific primers have generally been superior (higher mutation sensitivity) at detecting known single base mutations, while non-allele-specific primers with allele-specific blockers are generally applied for the detection of multiple closely clustered mutations (hotspots) or unknown mutation sequences.

Allele-specific PCR primers such as ARMS, ASB-PCR, castPCR generally possess an allele-specific nucleotide at the 3'-most position of the primer. The allele-specific PCR primers employ the discrimination of the DNA polymerase enzyme to specifically extend properly paired bases, but are limited by the fact that, when an incorrect DNA polymerase extension event does occur, the rare allele nucleotide that was a part of the primer becomes incorporated in the template, and subsequently amplification cycles become non-specific. The allele-specific PCR primers are only specific up to the first incorrect amplification event. Allele-specific detection with non-allele-specific primers requires precise denaturation temperature control, and restricted analysis of smaller sequences. The allele-specific detection with non-allele-specific primers has low mutation sensitivity and is more vulnerable to polymerase-introduced errors.

Thus, a composition of non-allele-specific primer and allele-specific blocker oligonucleotides with increased allele-specific amplification and improved mutation sensitivity is needed. Therefore, the development of new approaches suitable for the detection of a small amount of target in a large excess of variant is a prerequisite for the diagnostic applications. Thus, the present disclosure provides a composition of non-allele-specific primer and allele-specific blocker oligonucleotides to provide a more accurate and an efficient tool for disease diagnostic applications such as, cancer diagnosis and like.

**SUMMARY**

The present invention is set out in the appended claims, and relates to a method for amplification of a target sequence and an oligonucleotide composition for carrying out said method.

More specifically, the present invention provides a method for amplification of a target sequence comprising the steps of:

(a) obtaining a sample containing one or more copies of a first nucleic acid comprising a variant sequence and possibly containing at least one copy of a second nucleic acid comprising the target sequence, wherein the target sequence and variant sequence each comprise a homologous subsequence and a variable subsequence, wherein the variable subsequence comprises at least one nucleotide, and wherein the variable subsequence of the target sequence is a target-specific subsequence and the variable subsequence of the variant sequence is a non-target specific subsequence;

(b) introducing a blocker oligonucleotide to the sample comprising a first sequence comprising a target-neutral subsequence and a blocker variable subsequence, wherein the blocker variable subsequence is flanked on its 3' and 5' ends by the target-neutral subsequence and is continuous with the target-neutral subsequence, wherein the blocker variable subsequence divides the target-neutral subsequence into two portions, a portion to the 3' of the blocker variable subsequences and a portion to the 5' of the blocker variable subsequence, wherein the target-neutral subsequence is complementary to a portion of a homologous subsequence of both a first target nucleic acid and a first variant nucleic acid, wherein the blocker variable subsequence is complementary to a first variant subsequence of the first variant nucleic acid, and wherein the blocker oligonucleotide comprises a non-complementary sequence region at its 3' end, which prevents enzymatic extension and which is not complementary to a sequence continuous with the target-neutral subsequence; and

(c) introducing a first primer oligonucleotide to the sample, wherein the first primer oligonucleotide is sufficient to induce enzymatic extension, wherein the first primer oligonucleotide comprises a second sequence, wherein the second sequence is complementary to a second portion of the homologous subsequence, wherein the second sequence overlaps the target-neutral subsequence of the blocker oligonucleotide by at least 5 nucleotides such that the second sequence comprises an overlapping subsequence and a non-overlapping subsequence, and wherein the second sequence does not include any sequence complementary to the variable subsequence; wherein the second sequence of the first primer oligonucleotide yields a standard free energy of hybridization ($\Delta G°PT$) to the target sequence, and the first sequence of the blocker oligonucleotide yields a standard free energy of hybridization ($\Delta G°BT$) to the target sequence, which satisfies the following condition: +2 kcal/mol $\geq \Delta G°PT$ - $\Delta G°BT \geq$ -8 kcal/mol; and wherein the non-overlapping subsequence yields a standard free energy of hybridization ($\Delta G°3$) to the target sequence, which satisfies the following condition: -4 kcal/mol $\geq \Delta G°3 \geq$ -12 kcal/mol;

(d) introducing to the sample a DNA polymerase, nucleoside triphosphates, and one or more reagents necessary for polymerase-based nucleic acid amplification; and

(e) reacting the sample under conditions sufficient to achieve nucleic acid amplification.

The present invention also provides an oligonucleotide composition for carrying out said method, including (a) a first blocker oligonucleotide, (b) a first primer oligonucleotide, (c) a second blocker oligonucleotide, and (d) a second primer oligonucleotide.

(a) The first blocker oligonucleotide includes a first sequence having a target-neutral subsequence and a first blocker variable subsequence. The first blocker variable subsequence is flanked on its 3' and 5' ends by the first target-neutral subsequence and is continuous with the first target-neutral subsequence. The first blocker variable subsequence divides the first target-neutral subsequence into two portions, a portion to the 3' of the first blocker variable subsequences and a portion to the 5' of the first blocker variable subsequence. The first target-neutral subsequence is complementary to a first portion of a homologous subsequence of both a first target nucleic acid and a first variant nucleic acid. The first blocker variable subsequence is complementary to a first variant subsequence of the first variant nucleic acid. The first blocker oligonucleotide comprises a non-complementary sequence region at its 3' end, which prevents enzymatic extension and which is not complementary to a sequence continuous with the first target-neutral subsequence.

(b) The first primer oligonucleotide is sufficient to induce enzymatic extension for amplification of a desired first target sequence; wherein the first primer oligonucleotide includes a second sequence. The second sequence is complementary to a second portion of a homologous subsequence of both the first target nucleic acid and the first variant nucleic acid, and it overlaps with the 5' end of the target-neutral subsequence by at least 5 nucleotides such that the second sequence includes a first overlapping subsequence and a first non-overlapping subsequence. The second sequence does not include the first blocker variable subsequence.

The second sequence yields a standard free energy of hybridization ($\Delta G°PT$) to the target nucleic acid, and the first sequence yields a standard free energy of hybridization ($\Delta G°BT$) to the target nucleic acid, which satisfies the following condition: +2 kcal/mol $\geq \Delta G°PT$ - $\Delta G°BT \geq$ -8 kcal/mol; and the first non-overlapping subsequence of the first primer oligonucleotide yields a standard free energy of hybridization ($\Delta G°3$) to the target nucleic acid, which satisfies the following condition: -4 kcal/mol $\geq \Delta G°_3 \geq$ -12 kcal/mol.

(c) The second blocker oligonucleotide comprising a fourth sequence comprising a second target-neutral subsequence and a second blocker variable subsequence. The second blocker variable subsequence is flanked on its 3' and 5' ends by the second target-neutral subsequence and is continuous with the second target-neutral subsequence. The second blocker variable subsequence divides the second target-neutral subsequence into two portions, a portion to the 3' of the second blocker variable subsequences and a portion to the 5' of the second blocker variable subsequence. The second target-neutral subsequence is complementary to a first portion of a homologous subsequence of both a second target nucleic acid and a second variant nucleic acid. The second blocker variable subsequence is complementary to a second variant subsequence of the second variant nucleic acid, and the second blocker oligonucleotide comprises a non-complementary sequence region at its 3' end, which prevents enzymatic extension and which is not complementary to a sequence continuous with the second target-neutral subsequence.

(d) The second primer oligonucleotide is sufficient to induce enzymatic extension for amplification of a desired second target sequence, and it comprises a third sequence that is complementary to a second portion of a homologous subsequence of both the second target nucleic acid and the second variant nucleic acid, wherein the third sequence overlaps the second target-neutral subsequence by at least 5 nucleotides such that the third sequence comprises a second overlapping subsequence and a second non-overlapping subsequence. The third sequence does not include the second blocker variable subsequence.

The third sequence yields a standard free energy of hybridization ($\Delta G°PT$) to the second target nucleic acid, and wherein the fourth sequence yields a standard free energy of hybridization ($\Delta G°BT$) to the target nucleic acid, which satisfies the following condition: $+2$ kcal/mol $\geq \Delta G°PT - \Delta G°BT \geq -8$ kcal/mol. The second non-overlapping subsequence of the second primer oligonucleotide yields a standard free energy of hybridization ($\Delta G°3$) to the second target nucleic acid, which satisfies the following condition: $-4$ kcal/mol $\geq \Delta G°_3 \geq -12$ kcal/mol.

An advantage of the present invention is improved mutation sensitivity matching and/or exceeding allele-specific primer approaches.

Another advantage of the present invention is a simple 2-step thermal cycling protocol with significant (8°C) temperature robustness.

Yet another advantage of the present invention is to provide inexpensive DNA primer and blocker reagents without complex backbone or nucleotide modifications.

Yet another advantage of the present invention is compatibility with high fidelity enzymes.

Yet another advantage of the present invention is that the allele-specific blocker binds more strongly to the variant than to the target, so that the non-allele-specific primer more favourably displace blockers bound to target as compared to blockers bound to variant. The advantage of using non-allele-specific primers is that spuriously amplified variants result in amplification products (amplicons) bearing the variant sequence rather than the target sequence. Thus, specificity is compounded at every cycle.

## BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein.

FIG. 1 illustrates a schematic representation of a non-allele-specific primer and an allele-specific blocker for allele-specific amplification in accordance with the present invention.

FIG. 2 illustrates a schematic representation of a thermodynamic design of a primer and a blocker in accordance with the present invention.

FIG. 3 illustrates a schematic representation of calculation of $\Delta G°$ values from sequence in accordance with the present invention. FIG. 3 discloses SEQ ID NOS 1, 33, 1, 34, 2, 33, 2, and 34, respectively, in order of appearance.

FIG. 4 illustrates a schematic representation of rare allele detection in human genomic DNA in accordance with the Example 1. FIG. 4 discloses SEQ ID NOS 3-4, 35, 32, 36, and 32, respectively, in order of appearance.

FIG. 5 illustrates a schematic representation of rare temperature robustness in accordance with the Example 2. FIG. 5 discloses SEQ ID NOS 5-6 and 37-38, respectively, in order of appearance.

FIG. 6A illustrates a schematic representation of increased primer design flexibility in accordance with the Example 3.

FIG. 6B illustrates a schematic representation of the nucleic acid sequences of 4 sets of primer and blocker pairs that amplifies the human SNP rs3789806 in accordance with the Example 3. FIG. 6B discloses SEQ ID NOS 5-12 and 39-40, respectively, in order of appearance.

FIG. 7A illustrates a schematic representation of simulation of effects of $\Delta G°_{rxn1}$ with fixed $\Delta\Delta G°= 2$ kcal/mol in accordance with the Example 4.

FIG. 7B(I) illustrates a schematic representation of an experimental validation on SMAD7 rs4939827 in accordance with the Example 4. FIG. 7B(I) discloses SEQ ID NOS 3, 13-16, 4, 17, and 35-36, respectively, in order of appearance.

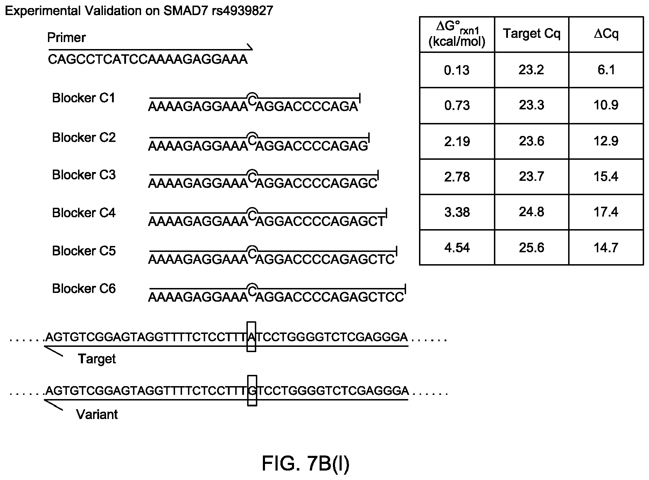

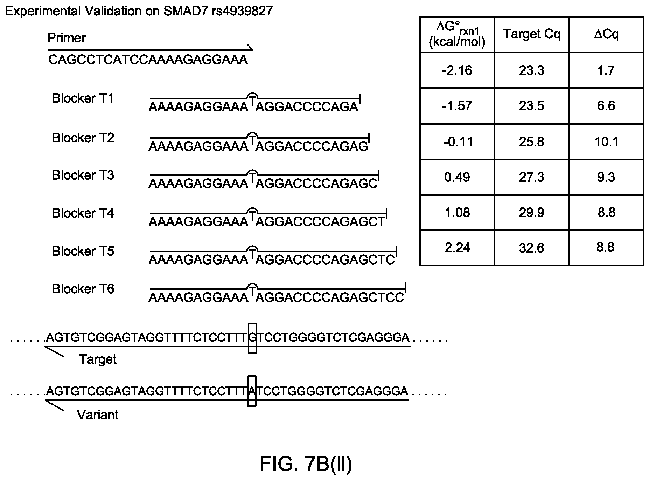

FIG. 7B(II) illustrates a schematic representation of an experimental validation on SMAD7 rs4939827 in accordance with the Example 4. FIG. 7B(II) discloses SEQ ID NOS 3, 18-23, 36, and 35, respectively, in order of appearance.

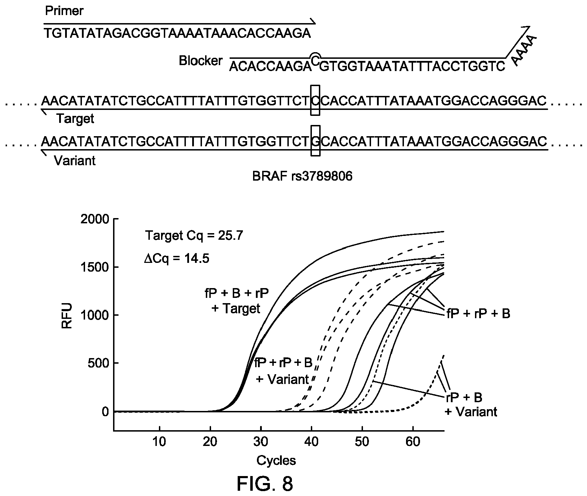

FIG. 8 illustrates a schematic representation of a blocker with 3' non-homologous region in accordance with the Example 5. FIG. 8 discloses SEQ ID NOS 5, 24, and 37-38, respectively, in order of appearance.

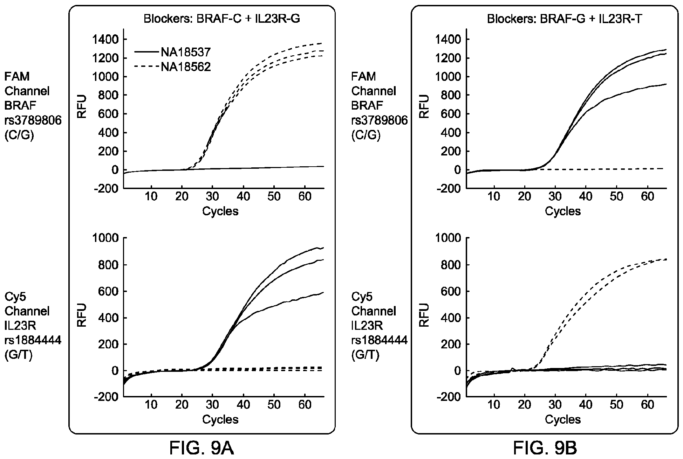

FIG. 9A illustrates a schematic representation of multiplexing using TaqMan® probes in accordance with the Example 6.

FIG. 9B illustrates a schematic representation of multiplexing using TaqMan® probes in accordance with the Example 6.

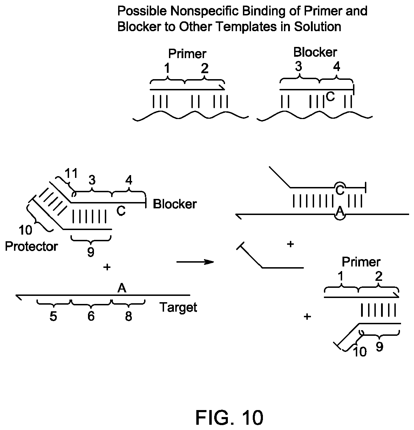

FIG. 10 illustrates a schematic representation of protectors for Primer and Blocker in accordance with the Example 7.

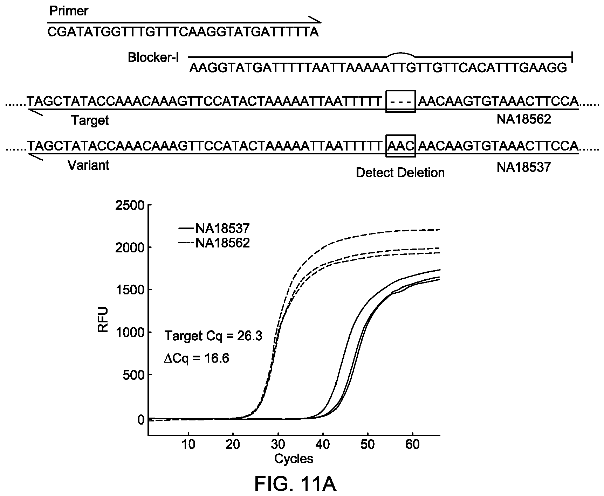

FIG. 11A illustrates a schematic representation of detection of deletion (STAG2 rs200841330) in accordance with the Example 8. FIG. 11A discloses SEQ ID NOS 25-26 and 41-42, respectively, in order of appearance.

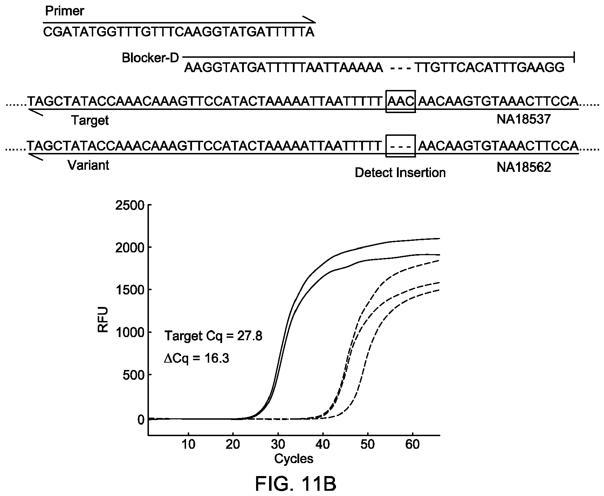

FIG. 11B illustrates a schematic representation of detection of insertion (STAG2 rs200841330) in accordance with the Example 8. FIG. 11B discloses SEQ ID NOS 25, 43, 42, and 41, respectively, in order of appearance.

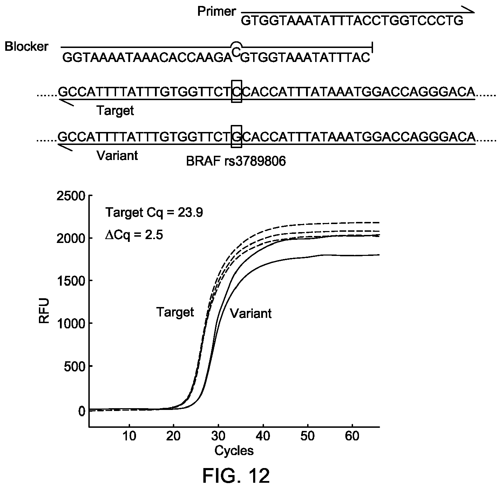

FIG. 12 illustrates a schematic representation of an upstream blocker in accordance with the Example 9. FIG. 12 discloses SEQ ID NOS 27-28 and 44-45, respectively, in order of appearance.

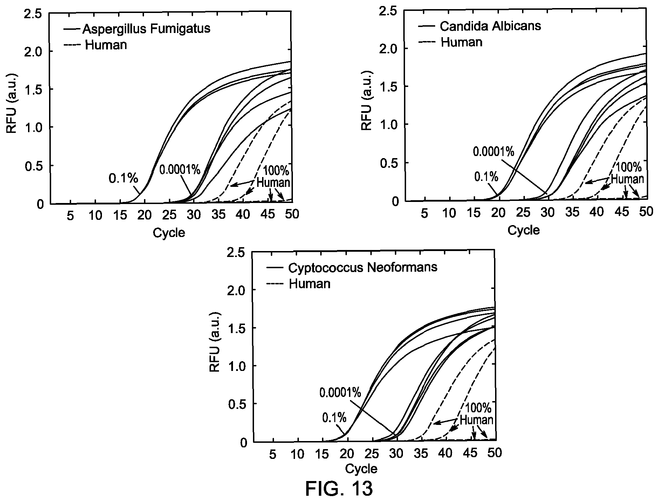

FIG. 13 illustrates a schematic representation of specific amplification of fungal ribosomal RNA-encoding DNA in accordance with the Example 10.

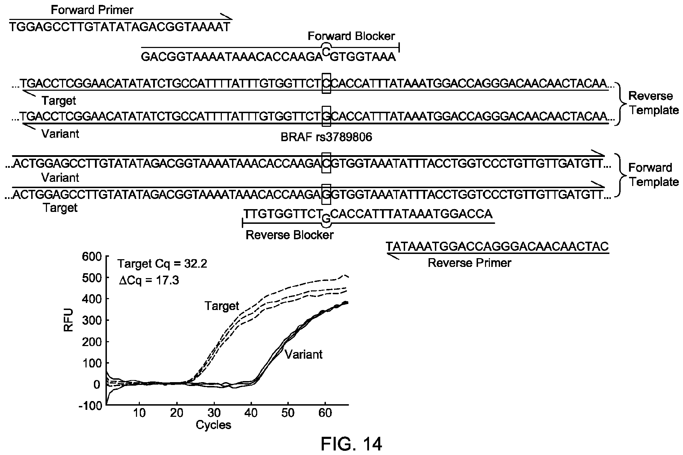

FIG. 14 illustrates a schematic representation of Dual NASP in accordance with the Example 11. FIG. 14 discloses SEQ ID NOS 29, 10, 46-49, and 30-31, respectively, in order of appearance.

## DETAILED DESCRIPTION OF THE INVENTION

The present invention will be described with respect to particular embodiments and with reference to certain drawings, but the invention is not limited thereto, but only by claims. The drawings described are only schematic and are non-limiting. In the drawings, the size of some of the elements may be exaggerated or distorted and not drawn on scale for illustrative purposes. Where the elements of the invention are designated as "a" or "an" in first appearance and designated as "the" or "said" for second or subsequent appearances unless something else is specifically stated.

The present invention now will be described more fully here later to with reference to the accompanying drawings, in which some, but not all embodiments of the invention are shown. Indeed, the invention may be embodied in many different forms and should not be construed as limited to the embodiments set forth herein, rather, these embodiments are provided so that this disclosure satisfies all the legal requirements.

*Definition*

Unless otherwise defined, all technical and scientific terms used herein have the same meaning as commonly understood by one of ordinary skill in the art.

As used herein, the term "blocker oligonucleotide" refers to at least one continuous strand of from about 12 to about 100 nucleotides in length and if so indicated herein, may further include a nucleotide sequence at its 3' end that prevents enzymatic extension during an amplification process such as polymerase chain reaction.

As used herein, the term "primer oligonucleotide" refers to a molecule comprising at least one continuous strand of from about 12 to about 100 nucleotides in length and sufficient to permit enzymatic extension during an amplification process such as polymerase chain reaction.

As used herein, the term "target-neutral subsequence" refers to a sequence of nucleotides that is complementary to a sequence in both a target nucleic acid and a variant nucleic acid. For example, a desired nucleic acid sequence to be targeted for amplification (target nucleic acid) may exist in a sample with a nucleic acid molecule having a predominantly homologous sequence with the target nucleic acid with the exception of a variable region (variant nucleic acid), such variable region in some instance being only a single nucleotide difference from the target nucleic acid. In this example, the target-neutral subsequence is complementary to at least a portion of the homologous sequence shared between the two nucleic acids, but not the variable region. Thus, as used herein, the term "blocker variable subsequence" refers to a nucleotide sequence of a blocker oligonucleotide which is complementary to the variable region of the variant nucleic.

As used herein, the term "overlapping subsequence" refers to a nucleotide sequence of at least 5 nucleotides of a primer oligonucleotide that is homologous with a portion of the blocker oligonucleotide sequence used in a composition as described herein. The overlapping subsequence of the primer oligonucleotide may be homologous to any portion of the target-neutral subsequence of the blocker oligonucleotide, whether 5' or 3' of the blocker variable subsequence. Thus, the term "non-overlapping subsequence" refers to the sequence of a primer oligonucleotide that is not the overlapping subsequence.

As used herein, the term "target sequence" refers to the nucleotide sequence of a nucleic acid that harbours a desired allele, such as a single nucleotide polymorphism, to be amplified, identified, or otherwise isolated. As used herein, the term "variant sequence" refers to the nucleotide sequence of a nucleic acid that does not harbour the desired allele. For example, in some instances, the variant sequence harbours the wild-type allele whereas the target sequence harbours the mutant allele. Thus, in some instance, the variant sequence and the target sequence are derived from a common locus in a genome such that the sequences of each may be substantially homologous except for a region harbouring the desired allele, nucleotide or group or nucleotides that varies between the two.

The present invention relates to an oligonucleotide composition for carrying out a method for amplification of a target sequence in accordance with the methods of the present invention, as set out in the appended claims.

The oligonucleotide composition includes (a) a first blocker oligonucleotide, (b) a first primer oligonucleotide, (c) a second blocker oligonucleotide, (d) a second primer oligonucleotide.

(a) The first blocker oligonucleotide includes a first sequence having a first target-neutral subsequence and a first blocker variable subsequence. The first blocker variable subsequence is flanked on its 3' and 5' ends by the first target-neutral subsequence and is continuous with the target-neutral subsequence. In other words, the first blocker variable subsequence divides the target-neutral subsequence into two portions, a portion to the 3' of the blocker variable subsequence and a portion to the 5'. The first target-neutral subsequence is complementary to a first portion of a homologous subsequence of both a first target nucleic acid and a first variant nucleic acid. The first blocker variable subsequence is complementary to a first variant subsequence of the first variant nucleic acid, and the first blocker oligonucleotide comprises a non-complementary sequence region at its 3' end, which prevents enzymatic extension and which is not complementary to a sequence continuous with the first target-neutral subsequence.

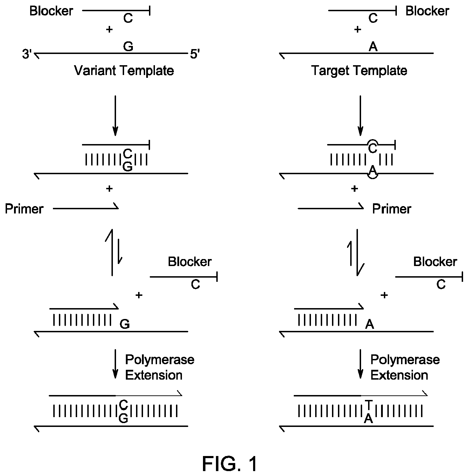

(b) The first primer oligonucleotide is sufficient to induce enzymatic extension for amplification of a desired first target sequence; wherein the first primer oligonucleotide includes a second sequence that is complementary to a second portion of a homologous subsequence of both the first target nucleic acid and the first variant nucleic acid. The second sequence overlaps with the target-neutral subsequence by at least 5 nucleotides such that the second sequence includes a first overlapping subsequence and a first non-overlapping subsequence. The second sequence does not include the first blocker variable subsequence. Thus, the first primer oligonucleotide can be characterized as a non-allele specific primer. The overlapping region can be homologous to a portion of the target-neutral subsequence of the blocker oligonucleotide on either the 5' side of the blocker variable subsequence or on the 3' side of the blocker variable subsequence. As shown in FIG. 1, the primer oligonucleotide overlapping subsequence is homologous with the target-neutral subsequence of the blocker on the 5' side of the blocker variable subsequence (denoted as "C" on the blocker). Here, the target-neutral subsequence is the portions of the blocker on either side of the blocker variable subsequence C. FIG. 12 depicts one example where the overlapping subsequence of the primer is homologous with a portion of target-neutral subsequence of the blocker that is 3' of the blocker variable subsequence C.

(c) The second blocker oligonucleotide is as defined further below.

(d) The second primer oligonucleotide is as defined further below.

In an embodiment of the present invention, the first blocker oligonucleotide further includes a non-complementary sequence region at or near the 3' end, which prevents enzymatic extension.

The second sequence yields a standard free energy of hybridization ($\Delta G^{\circ}_{PT}$) and the first sequence yields a standard free energy of hybridization ($\Delta G^{\circ}_{BT}$), which satisfies the following condition:

$$+2 \text{ kcal/mol} \geq \Delta G^{\circ}_{PT} - \Delta G^{\circ}_{BT} \geq -8 \text{ kcal/mol}$$

The non-overlapping subsequence yields a standard free energy of hybridization ($\Delta G^{\circ}_3$), which satisfies the following condition:

$$-4 \text{ kcal/mol} \geq \Delta G^{\circ}_3 \geq -12 \text{ kcal/mol}$$

In an instance, the first primer oligonucleotide comprises a second sequence that overlaps with the 5' end of the target-neutral subsequence by about 5 nucleotides to about 40 nucleotides. In another instance, the second sequence overlaps with the 5' end of the target-neutral subsequence by about 7 nucleotides to about 30 nucleotides.

In an instance, the concentration of the first blocker oligonucleotide is about 2 to about 10,000 times greater than the concentration of the first primer oligonucleotide. In another instance, the concentration of the first blocker oligonucleotide is about 5 to about 1,000 times greater than the concentration of the first primer oligonucleotide.

The oligonucleotide composition further includes the second primer oligonucleotide sufficient to induce enzymatic extension for amplification of a desired second target sequence. The second primer oligonucleotide includes a third sequence that is complementary to a second portion of a homologous subsequence of both the second target nucleic acid and the second variant nucleic acid. The third sequence overlaps the second target-neutral subsequence by at least 5 nucleotides such that the third sequence comprises a second overlapping subsequence and a second non-overlapping subsequence, and the third sequence does not include the second blocker variable subsequence.

The oligonucleotide composition further includes a second blocker oligonucleotide. The second blocker oligonucleotide includes a fourth sequence having a second target-neutral subsequence and a second blocker variable subsequence. The second blocker variable subsequence may have the same sequence as the non-target specific subsequence of the target nucleic acid given that it is typically designed to bind the antisense sequence of the target. The second blocker variable subsequence is flanked on its 3' and 5' ends by the second target-neutral subsequence and is continuous with the second target-neutral subsequence. The second blocker variable subsequence divides the second target-neutral subsequence into two portions, a portion to the 3' of the second blocker variable subsequences and a portion to the 5' of the second blocker variable subsequence. The second target-neutral subsequence is complementary to a first portion of a homologous subsequence of both a second target nucleic acid and a second variant nucleic acid. The second blocker variable subsequence is complementary to a second variant subsequence of the second variant nucleic acid, and the second blocker oligonucleotide comprises a non-complementary sequence region at its 3' end, which prevents enzymatic extension and is not complementary to a sequence continuous with the second target-neutral subsequence. The third sequence overlaps with the 5' end of the second target-neutral subsequence by at least 5 nucleotides such that the third sequence includes a second overlapping subsequence and a second non-overlapping subsequence.

In an embodiment of the present invention, the second blocker oligonucleotide further includes a second non-complementary sequence region at or near the 3' end, which prevents enzymatic extension.

The third sequence yields a standard free energy of hybridization ($\Delta G°_{PT2}$) and the fourth sequence yields a standard free energy of hybridization ($\Delta G°_{BT2}$), which satisfies the following condition:

$$+2 \text{ kcal/mol} \geq \Delta G°_{PT2} - \Delta G°_{BT2} \geq -8 \text{ kcal/mol}$$

The second non-overlapping subsequence yields a standard free energy of hybridization ($\Delta G°_6$), which satisfies the following condition:

$$-4 \text{ kcal/mol} \geq \Delta G°_6 \geq -12 \text{ kcal/mol}.$$

In one instance, the third sequence overlaps with the 5' end of the second target-neutral subsequence by about 5 nucleotides to about 40 nucleotides. In another instance, the third sequence overlaps with the 5' end of the second target-neutral subsequence by about 7 nucleotides to about 30 nucleotides.

In any of the above embodiments, the primer oligonucleotide and blocker oligonucleotide may each be from about 12 nucleotides in length to about 100 nucleotides in length. In any of the above embodiments, the primer oligonucleotide and blocker oligonucleotide may each be from about 15 nucleotides in length to about 90 nucleotides in length. In any of the above embodiments, the primer oligonucleotide and blocker oligonucleotide may each be from about 20 nucleotides in length to about 80 nucleotides in length. In any of the above embodiments, the primer oligonucleotide and blocker oligonucleotide may each be from about 20 nucleotides in length to about 70 nucleotides in length. In any of the above embodiments, the primer oligonucleotide and blocker oligonucleotide may each be from about 20 nucleotides in length to about 60 nucleotides in length. In any of the above embodiments, the primer oligonucleotide and blocker oligonucleotide may each be 21, 22, 23, 24, 26, 27, 28, 29, 30, 31, 32, 33, 34, 35, 36, 37, 38, 39, 40, 41, 42, 43, 44, 45, 46, 47, 48, 49, or 50 nucleotides in length.

In an instance, the concentration of the second blocker oligonucleotide is about 2 to about 10,000 times greater than the concentration of the second primer oligonucleotide. In another instance, the concentration of the second blocker oligonucleotide is about 5 to about 1,000 times greater than the concentration of the second primer oligonucleotide.. In another instance, the concentration of the second blocker oligonucleotide is about 10 to about 900 times greater than the concentration of the second primer oligonucleotide. In another instance, the concentration of the second blocker oligonucleotide is about 20 to about 800 times greater than the concentration of the second primer oligonucleotide.. In another instance, the concentration of the second blocker oligonucleotide is about 30 to about 700 times greater than the concentration of the second primer oligonucleotide. In another instance, the concentration of the second blocker oligonucleotide is about 40 to about 600 times greater than the concentration of the second primer oligonucleotide. In another instance, the concentration of the second blocker oligonucleotide is about 50 to about 500 times greater than the concentration of the second primer oligonucleotide. In another instance, the concentration of the second blocker oligonucleotide is about 60 to about 400 times greater than the concentration of the second primer oligonucleotide. In another instance, the concentration of the second blocker oligonucleotide is about 70 to about 300 times greater than the concentration of the second primer oligonucleotide. In another instance, the concentration of the second blocker oligonucleotide is about 80 to about 200 times greater than the concentration of the second primer oligonucleotide. In another instance, the concentration of the second blocker oligonucleotide is about 90 to about 100 times greater than the concentration of the second primer oligonucleotide.

In yet another embodiment of the present invention, the oligonucleotide composition further includes a reagent necessary for polymerase chain reaction.

In yet another embodiment of the present invention, the oligonucleotide composition further includes a plurality of nucleoside triphosphates.

In yet another embodiment of the present invention, the oligonucleotide composition further includes a DNA polymerase or an RNA polymerase.

In yet another embodiment of the present invention, the oligonucleotide composition further includes a reagent necessary for polymerase chain reaction, a plurality of nucleoside triphosphates, a DNA polymerase.

In yet another embodiment of the present invention, the blocker variable subsequence is a single nucleotide.

The present invention further relates to a method for amplification of a target sequence, as set out in the appended claims. The method includes steps of (a) obtaining a sample containing one or more copies of a first nucleic acid having a variant sequence and possibly containing at least one copy of a second nucleic acid; wherein the second nucleic acid has the target sequence. The target sequence and variant sequence each has a homologous subsequence and a variable subsequence. The variable subsequence further includes at least one nucleotide, but may include 2-5 nucleotides. Furthermore, the variable subsequence of the target sequence is a target-specific subsequence and the variable subsequence of the variant sequence is a non-target specific subsequence.

The method further comprises the step of (b) introducing a blocker oligonucleotide to the sample; wherein the blocker oligonucleotide includes a first sequence having a target-neutral subsequence and a blocker variable subsequence. The target-neutral subsequence is complementary to a portion of the homologous subsequence of both a first target nucleic acid and a first variant nucleic acid, and the blocker variable subsequence is complementary to a first variant subsequence of the first variant nucleic acid. Further, the blocker variable subsequence is flanked on its 3' and 5' ends by the target-neutral subsequence and is continuous with the target-neutral subsequence, such that a the blocker variable subsequence divides the target-neutral subsequence into two portions. The blocker oligonucleotide comprises a non-complementary sequence region at its 3' end, which prevents enzymatic extension and which is not complementary to a sequence continuous with the target-neutral subsequence.

The method further comprises the step of (c) introducing a first primer oligonucleotide to the sample. The first primer oligonucleotide is sufficient to induce enzymatic extension. The first primer oligonucleotide includes a second sequence. Further, the second sequence is complementary to a second portion of the homologous subsequence, and overlaps the target-neutral subsequence of the blocker oligonucleotide by at least 5 nucleotides such that the second sequence includes an overlapping subsequence and a non-overlapping subsequence. The second sequence does not include any sequence complementary to the variable subsequence.

The method further comprises the step of (d) introducing to the sample a DNA polymerase, nucleoside triphosphates, and one or more reagents necessary for polymerase-based nucleic acid amplification; and then (e) reacting the sample under conditions sufficient to achieve nucleic acid amplification.

In an embodiment of the present invention, the blocker oligonucleotide further includes a non-complementary sequence region at or near the 3' end, which prevents enzymatic extension.

The second sequence of the first primer oligonucleotide yields a standard free energy of hybridization ($\Delta G°_{PT}$) to the target sequence, and the first sequence of the blocker oligonucleotide yields a standard free energy of hybridization ($\Delta G°_{BT}$) to the target sequence, which satisfies the following condition:

$$+2 \text{ kcal/mol} \geq \Delta G°_{PT} - \Delta G°_{BT} \geq -8 \text{ kcal/mol}$$

The non-overlapping subsequence yields a standard free energy of hybridization ($\Delta G°_3$) to the target sequence, which satisfies the following condition:

$$-4 \text{ kcal/mol} \geq \Delta G°_3 \geq -12 \text{ kcal/mol}$$

In one instance, the second sequence overlaps with the 5' end of the target-neutral subsequence of the blocker oligonucleotide by about 5 nucleotides to about 40 nucleotides (i.e., the "overlapping subsequence"). In other words, the second sequence comprises an overlapping subsequence that is homologous with 5 nucleotides to about 40 nucleotides of the target-neutral subsequence of the blocker oligonucleotide that is on the 5' side of the blocker variable subsequence of the blocker. In another instance, the second sequence overlaps with the 5' end of the target-neutral subsequence of the blocker oligonucleotide by about 7 nucleotides to about 30 nucleotides. In yet another instance, the second sequence comprises an overlapping subsequence that is homologous to the portion of the target neutral subsequence of the blocker oligonucleotide that is on the 3' side of the blocker variable subsequence of the blocker.

In one instance, the concentration of the blocker oligonucleotide introduced into the sample is about 2 to about 10,000 times greater than the concentration of the first primer oligonucleotide introduced into the sample. In another instance, the concentration of the blocker oligonucleotide introduced into the sample is about 5 to about 1,000 times greater than the concentration of the first primer oligonucleotide introduced into the sample. In another instance, the concentration of the blocker oligonucleotide introduced into the sample is about 10 to about 500 times greater than the concentration of the first primer oligonucleotide introduced into the sample. In another instance, the concentration of the blocker oligonucleotide introduced into the sample is about 20 to about 250 times greater than the concentration of the first primer oligonucleotide introduced into the sample. In another instance, the concentration of the blocker oligonucleotide introduced into the sample is about 40 to about 125 times greater than the concentration of the first primer oligonucleotide introduced into the sample. In another instance, the concentration of the blocker oligonucleotide introduced into the sample is about 50 to about 100 times greater than the concentration of the first primer oligonucleotide introduced into the sample. In another instance, the concentration of the blocker oligonucleotide introduced into the sample is about 300 to about 400 times greater than the concentration of the first primer oligonucleotide introduced into the sample. In another instance, the concentration of the blocker oligonucleotide introduced into the sample is about 500 to about 600 times greater than the concentration of the first primer oligonucleotide introduced into the sample.

In another embodiment of the present invention, the DNA polymerase is a thermostable DNA polymerase.

In yet another embodiment of the present invention, the method further includes the conditions sufficient to achieve nucleic acid amplification by exposing the sample to at least 10 cycles. Each cycle has at least 2 different temperature exposures, one temperature exposure of at least 85°C, and one temperature exposure of no more than 75°C.

In yet another embodiment of the present invention, the method further includes the step of introducing to the sample an enzyme selected from the group consisting of a nicking enzyme, a recombinase, a helicase, a RNAse a reverse transcriptase, or any combination thereof.

In yet another embodiment of the present invention, the method further includes a step of introducing a second primer oligonucleotide into the sample. The second primer oligonucleotide includes a third sequence. Further, the third sequence does not overlap with the variable subsequence. Furthermore, the third sequence is target-neutral and is sufficient to use with the first primer oligonucleotide to amplify a region of a nucleic acid having the target sequence.

In yet another embodiment of the present invention, the method further includes a step of introducing a second blocker oligonucleotide to the sample. The second blocker oligonucleotide includes a fourth sequence; herein the fourth sequence has a second target-neutral subsequence and a second blocker variable subsequence. Further, the second blocker variable subsequence is the complementary sequence to the blocker variable subsequence. Furthermore, the second blocker variable subsequence is flanked on its 3' and 5' ends by the second target-neutral subsequence and is continuous with the second target-neutral subsequence. In addition, the third sequence overlaps with the 5' end of the second target-neutral subsequence by at least 5 nucleotides such that the third sequence includes a second overlapping subsequence and a second non-overlapping subsequence.

In yet another embodiment of the present invention, the second blocker oligonucleotide further includes a second non-complementary sequence region at or near the 3' end, which prevents enzymatic extension.

The third sequence yields a standard free energy of hybridization ($\Delta G^\circ_{PT2}$) and the fourth sequence yields a standard free energy of hybridization ($\Delta G^\circ_{BT2}$), which satisfies the following condition:

$$+2 \text{ kcal/mol} \geq \Delta G^\circ_{PT2} - \Delta G^\circ_{BT2} \geq -8 \text{ kcal/mol}$$

The second non-overlapping subsequence yields a standard free energy of hybridization ($\Delta G^\circ_6$), which satisfies the following condition:

$$-4 \text{ kcal/mol} \geq \Delta G^\circ_6 \geq -12 \text{ kcal/mol}$$

In an instance, the third sequence overlaps with the 5' end of the second target-neutral subsequence by about 5 nucleotides to about 40 nucleotides. In another instance, the third sequence overlaps with the 5' end of the second target-neutral subsequence by about 7 nucleotides to about 30 nucleotides.

In an instance, the concentration of the second blocker oligonucleotide introduced into the sample is about 2 to about 10,000 times greater than the concentration of the second primer oligonucleotide introduced into the sample. In another instance, the concentration of the second blocker oligonucleotide introduced into the sample is about 5 to about 1,000 times greater than the concentration of the second primer oligonucleotide introduced into the sample.

In yet another embodiment of the present invention, the method for amplification of a target sequence further includes a step of removing an aliquot from the sample; and repeating steps (b) through (e) defined above.

FIG. 1 illustrates a schematic representation of the non-allele-specific primer and the allele-specific blocker for allele-specific amplification. A nucleic acid reagent mixture comprises two oligonucleotide species, a primer (primer oligonucleotide) and a variant specific blocker (blocker oligonucleotide), which act together to enable reliable rare target amplification in conjunction with a polymerase-based amplification method. The blocker may be modified at the 3' end with a non-extensible modification, such as a dideoxynucleotide or a 3-carbon linker. The sequences of the primer and blocker are rationally designed based on the thermodynamics of their hybridization to the target nucleic acid sequence and the variant nucleic acid sequence. In some embodiments, the blocker is present in a significantly higher concentration than the primer, so that the preponderance of the target and the variant nucleic acid sequences bind to blocker before binding to primer. The primer binds transiently to the blocker-target or blocker-variant molecules, and possesses a probability for displacing the blocker in binding to the target or variant. Because the blocker sequence is specific to the variant target, its displacement from the variant is less thermodynamically favourable than its displacement from the target. Thus, the non-allele-specific primer amplifies the target sequence with higher yield/efficiency than it amplifies the variant sequence. The non-allele-specific nature of the primer means that spuriously amplified variant sequence bear the variant allele, rather than the target allele, so that subsequent amplification cycles also exhibit amplification bias in favour of the target.

High concentration of the variant-specific blocker results in higher probability of blocker binding to both the target and the variant first, before the primer has an opportunity to bind. The primer initiates binding, to both the target-blocker or variant-blocker complex, via a unique region not overlapping with the blocker, and subsequently possibly displaces the blocker via a process of enzyme-free strand displacement.

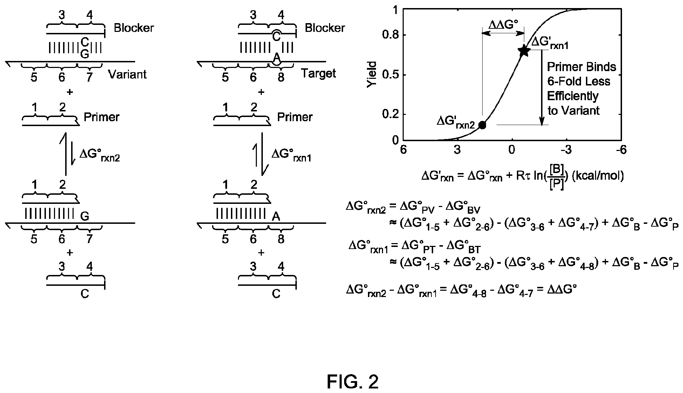

FIG. 2 illustrates a schematic representation of a thermodynamic design of a primer and a blocker. The sequences of the primer and the blocker are rationally designed to achieve desirable reaction thermodynamics. Here, the primer, the blocker, the target, and the variant nucleic acid sequences are subdivided into regions comprising continuous nucleotides, denoted by the numbers 1 through 8 in Fig. 2. The standard free energy of hybridization between the primer and the target ($\Delta G°_{PT}$) and the standard free energy of hybridization between the blocker and the target ($\Delta G°_{BT}$) satisfies:

$$+2 \text{ kcal/mol} \geq \Delta G°_{PT} - \Delta G°_{BT} \geq -8 \text{ kcal/mol}$$

The primer and blocker systems that satisfy the above inequality generate a large difference in the hybridization yields between primer bound to the target and primer bound to the variant in each cycle, resulting in a target-specific amplification.

The process of enzyme-free strand displacement is guided by the relative thermodynamics of the primer binding versus the blocker binding. The blocker hybridizes more favourably to the variant (standard free energy of binding $\Delta G°_{BV}$) than to the target (standard free energy of binding $\Delta G°_{BT}$, $\Delta G°_{BT} > \Delta G°_{BV}$ because more negative $\Delta G°$ indicates stronger favourability). In another embodiment of the present invention, the primer hybridizes equally favourably to the variant (standard free energy of binding $\Delta G°_{PV}$) as to the target (standard free energy of binding $\Delta G°_{PT}$, $\Delta G°_{PV} = \Delta G°_{PT}$).

The reaction of the primer displacing the blocker in binding to the target, BT + P <=> PT + B, has a standard free energy $\Delta G°_{rxn1} = \Delta G°PT - \Delta G°_{BT}$, which is more negative (more favourable) than that of the reaction of the primer displacing the blocker in binding to the variant, BV + P ⇔ PV + B, which has standard free energy $\Delta G°_{rxn2} = \Delta G°_{PV} - \Delta G°_{BV}$. For good performance of the present invention, the primer and blocker usually should be designed so that $\Delta G°_{rxn1} \leq 0$ and $\Delta G°_{rxn2} \geq 0$. However, because the relative concentrations of the blocker and the primer can influence the equilibrium distribution and effective thermodynamics of the reaction, the $\Delta G°_{rxn1}$ and $\Delta G°_{rxn2}$ guidelines are not absolute.

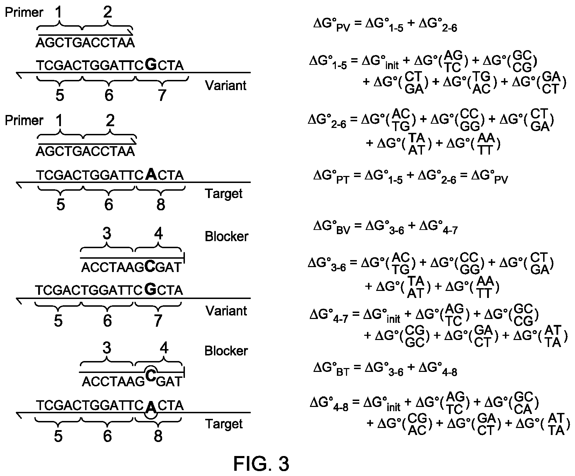

FIG. 3 illustrates a schematic representation of calculation of $\Delta G°$ values from sequence. There exist different conventions for calculating the $\Delta G°$ of different region interactions; shown in FIG. 3 are exemplary energy calculations based on the nearest neighbour model. The $\Delta G°_{1-5}$, $\Delta G°_{4-7}$, and $\Delta G°_{4-8}$ terms further include the standard free energy of hybridization initiation ($\Delta G°_{init}$), and the extra base stack adjoining the sixth region. Standard free energies, $\Delta G°$, calculated using other methods may result in the same $\Delta G°_{PT}$, $\Delta G°_{PV}$, $\Delta G°_{BT}$, and $\Delta G°_{BV}$ values, though the thermodynamics of individual regions (e.g. $\Delta G°_{3-6}$) may differ. The illustrative examples are intended to show the method of $\Delta G°$ calculation used in the present invention, and are not intended to be suggestive of sequences for an assay; the listed sequences may be too short to stably bind. The calculation of $\Delta G°_{PT}$, $\Delta G°_{PV}$, $\Delta G°_{BT}$, and $\Delta G°_{BV}$ from the primer sequence, blocker sequence, target sequence, variant sequence, operational temperature, and operational buffer conditions are known to those skilled in the art.

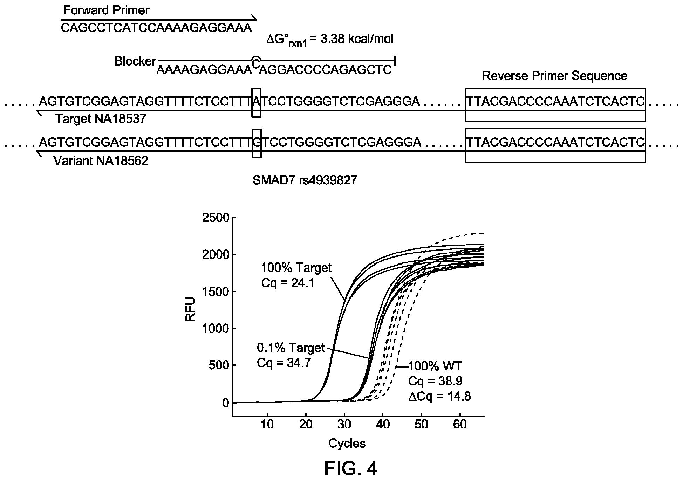

FIG. 4 shows a probe and a blocker design of the present invention and the subsequences around SMAD7 gene locus of two human repository samples NA18537 and NA18562. The design of a primer/blocker set designed to specifically amplify the A variant of the SMAD7 single nucleotide polymorphism (SNP). The blocker is designed to be perfectly complementary to the variant template bearing a G-variant of the SMAD7 SNP, and is functionalized at the 3' end with a 3-carbon linker (C3) that prevents enzymatic extension by the Taq DNA polymerase. A reverse primer binds to the 3' of the forward primer, and is not specific to either allele. For the sake of explanation of terminology used to describe various subsequences of the primar and blocker, the oligonucleotides can be described as follows. The blocker oligonucleotide comprises a target-neutral subsequence which includes the sequence to the 5' and 3' of the cytosine nucleotide that is depicted out of alignment with the remainder of the sequence - the cytosine nucleotide in this instance represents the blocker variable subsequence of the blocker. The overlapping subsequence of the primer oligonucleotide comprises sequence that is homologous to the blocker sequence on the 5' side of the blocker variable subsequence (the cytosine).

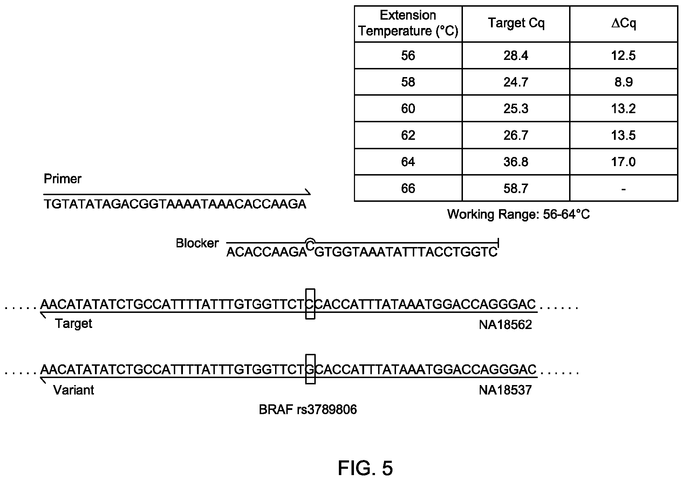

FIG. 5 shows the performance of a primer-blocker pair targeting human SNP rs3789806 (C/G) C allele at annealing/extension temperature ranging from 56°C to 66°C.

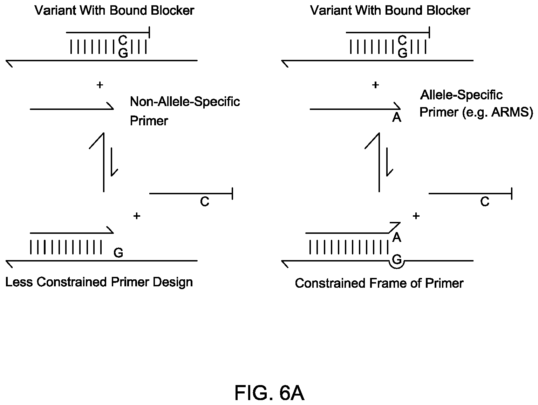

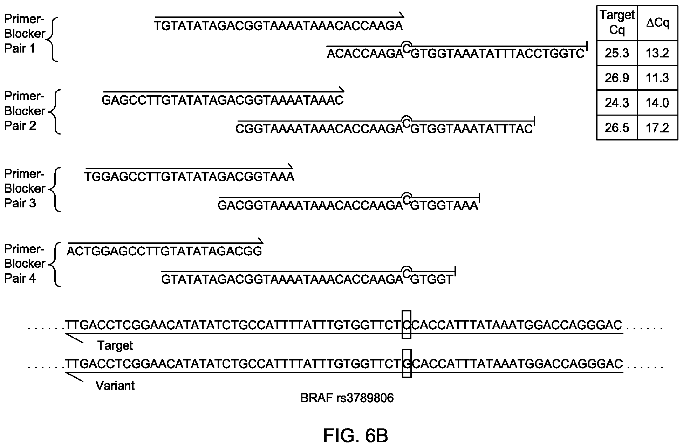

FIG. 6A and FIG. 6B shows primer designs with more design space to avoid undesired interactions.

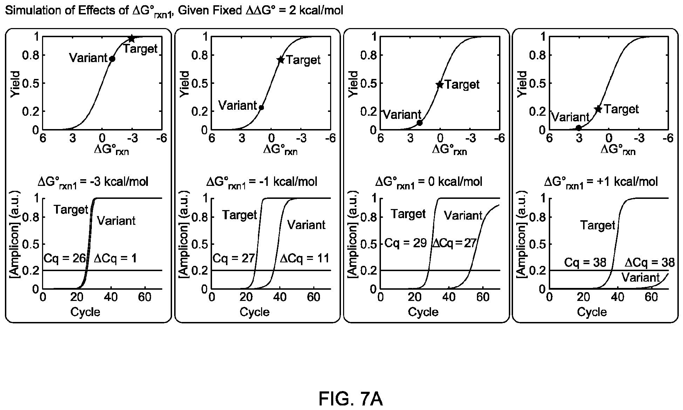

FIG. 7A, and FIG. 7B shows controlling the allele-specific PCR behaviour via design of $\Delta G^\circ_{rxn1}$. Simulations in FIG. 7A suggest that when the primer and blocker are designed so that $\Delta G^\circ_{rxn1}$ is more positive, $\Delta Cq$ values are greater. However, at more positive values of $\Delta G^\circ_{rxn1}$, the amplification of the target is also slowed, resulting in a larger value of Cq. Simulations assume a fixed $\Delta\Delta G^\circ$ of 2 kcal/mol. Top sub graphs show the predicted effects of the different values of $\Delta G^\circ_{rxn1}$ (stars) and $\Delta G^\circ_{rxn2}$ (dots) on the hybridization yields. The bottom subgraphs show simulated amplification curve of the target and the variant for each primer and blocker pair. In all cases depicted, the concentration ratio of blocker to primer is 20:1. FIG. 7B(I) and FIG. 7B(II) show experimental results of $\Delta G^\circ_{rxn1}$ effects on amplification selectivity. Different $\Delta G^\circ_{rxn1}$ values were achieved via lengthening or shortening the 3' end of the blocker. As expected, more positive $\Delta G^\circ_{rxn1}$ results in larger Cq for both the target and the variant. We observed an optimal intermediate $\Delta G^\circ_{rxn1}$ resulting in largest $\Delta Cq$ and relatively small Cq delay (Cq < 28) for the target. Unlike the simulations, $\Delta Cq$ did not increase monotonically with $\Delta G^\circ_{rxn1}$. The formation of primer-dimers and nonspecific amplification produces a Cq ceiling.

The kinetic simulations of the PCR process expressed as:

$$Tf_{n+1} = Tf_n + P_n \bullet [P_n / (P_n + B_n) + Y(\Delta G^\circ_{rxn1}) \bullet B_n / (P_n + B_n)] \bullet Tr_n$$

$$Tr_{n+1} = Tr_n + R_n \bullet Tf_n$$

$$Vf_{n+1} = Vf_n + P_n \bullet [P_n / (P_n + B_n) + Y(\Delta G^\circ_{rxn2}) \bullet B_n / (P_n + B_n)] \bullet Vr_n$$

$$Vr_{n+1} = Vr_n + R_n \bullet Vf_n$$

where Tfn is the normalized concentration of the forward strand of the target at cycle n, Trn is the normalized concentration of the reverse strand of the target at cycle n, Vfn is the normalized concentration of the forward strand of the variant at cycle n, Vrn is the normalized concentration of the forward strand of the variant at cycle n, Pn is the normalized concentration of the forward primer at cycle n, Bn is the normalized concentration of the blocker at cycle n, Rn is the normalized concentration of the reverse primer at cycle n, and $Y(\Delta G^\circ)$ is the hybridization yield of a displacement reaction given $\Delta G^\circ$ reaction standard free energy. Simulation results are shown in FIG. 7A.

FIG. 8 shows the blocker with a non-homologous sequence at the 3' end rather than a non-extensible chemical functionalization. Because the non-homologous sequences do not bind to the downstream region of either the target or the variant, the non-homologous region effectively prevents enzymatic extension. The target Cq and $\Delta Cq$ values are similar to those shown in Example 1, indicating that like functional groups such as a 3-carbon spacer or a didexoynucleotide, a non-homologous sequence at the 3' end can effectively prevent enzymatic extension Here fP, rP, and B represent the forward primer, the reverse primer, and the blocker (for the forward primer), respectively.

In standard blocking PCR, only the forward primer is biased to amplify the target sequence; the reverse primer amplifies both the target and the variant roughly equally. Amplification specificity can be further improved (with roughly a quadratic improvement in mutation sensitivity) if both the forward and the reverse primers were biased to amplify only the target sequence. To achieve dual target-specific amplification, the forward and reverse primers are designed to be separated by a short distance, so that they overlap with a variant-specific blocker. In the limit, the primer-binding regions are separated by a single nucleotide whose identity varies between the target and the variant. The forward and reverse variant-specific blockers are partially complementary to each other because they bind to complementary strands of the target, and both span the variation region. The blockers are designed so that, despite their partial complementarity, at the operational conditions, the majority of blocker molecules are not hybridized to each other.

FIG. 9A and FIG. 9B, each shows a primer-blocker systems designed for human BRAF rs3789806 and IL23R rs1884444 SNPs were multiplexed in the same reaction. TaqMan® probes targeting the corresponding downstream of the blocker binding regions were designed and used as the readout mechanism.

FIG. 10 shows nonspecific binding of primer or blocker to other primers, blockers, or templates results in nonspecific amplification. The lower panel provides a design of a protector that suppresses the nonspecific binding of the primer and blocker. Protector oligonucleotides are partially complementary to the primer and blocker sequences. The presence of a stoichiometric excess of protectors result in both the primer and the blocker being partially double-stranded. The blocker possesses a new region 11, whose sequence is non complementary to region 5 on the nucleic acid sequence, and the protector possesses a new region 10, whose sequence is complementary to region 11.

FIG. 11A and FIG. 11B, each shows primer and blocker designs for detecting a deletion and an insertion. The primer is non-target-specific, this method can also be used to detect deletion or insertion with uncertain number of bases or uncertain position.

FIG. 12 shows an inferior implementation of the present invention by placing the primer at the 3' of the SNP position and blocker at the 5' of the primer. This implementation generates hybridization specificity only in the first cycle, so the ∆Cq is smaller than the preferred design.

FIG. 13 demonstrates selective amplification of fungal 18S DNA subsequences of 3 pathogenic fungi species in large excess of homologous human DNA. Because fungal 18S subsequences and its homologous human subsequence differ in multiple regions, we designed corresponding blocker species for both forward primer and reverse primer to maximize amplification specificity.

FIG. 14 describes the use of target-specific amplification for both the forward and the reverse primer, by using two sets of variant-allele blockers, one for each primer. The blockers are partially complementary to each other, but at the operational conditions are not predominately hybridized to one another. The blockers and primers are designed so that the forward primer cannot extend off the reverse blocker, and the reverse primer cannot extend off the forward blocker. FIG. 14 shows preliminary experimental results.

The primer and blocker combinations may be applied to other enzymatic amplification assays for nucleic acids, including but not limited to Nicking Enzyme Amplification Reaction (NEAR), Loop-mediated Isothermal Amplification (LAMP), and Rolling Circle Amplification (RCA).

The present invention is particularly suitable for the detection of a small amount of target in a large excess of variant. For cancer diagnostics applications of the present invention, the variant may refer to the wildtype DNA sequence, and the target may refer a cancer DNA sequence. For infectious disease diagnostics applications of the present invention, the variant may refer to the pathogen DNA sequence, and the target may refer to a homologous human DNA sequence.

*Examples*

Example 1

FIG. 4 shows an Example 1 of the present invention for the probe and the blocker design and the subsequences around SMAD7 gene locus of two human repository samples NA18537 and NA18562. Healthy human single nucleotide polymorphisms (SNP) rs4939827 (C/T) in SMAD7 gene locus were chosen for proof-of-concept validations. T allele as target and C allele variant were arbitrarily designated. Human repository genomic DNA samples NA18562 (homozygous of C allele) and NA18537 (homozygous of T allele) were purchased from Coriell®, and the corresponding nucleotide sequences were obtained from 1000 Genomes website. To prevent unintended polymerization, the blocker was modified with a C3 spacer at the 3' end. qPCR experimental results distinguished 0.1% target (99.9% variant) from 0% target (100% variant). The 0.1% target sample was prepared by mixing 0.1% NA18537 with 99.9% NA18562. The average difference of quantification cycle (∆Cq) between 100% variant and 100% target was 14.8, and that between 100% variant (denoted as "WT" in the graph) and 0.1% target was 4.2. All experiments were performed in a 96-well plate in the Bio-Rad CFX96™ qPCR machine. In each well, 200 nM primer, 2 μM blocker, and 20 ng human genomic DNA were mixed in the Bio-rad iTaq™ SYBR® Green Supermix. After a 3 min initiation process at 95°C, 65 cycles of denaturing step at 95°C for 10 seconds and annealing/extension step at 60°C for 30 seconds were performed. Example experiments shown in FIG. 5-12 used a similar protocol.

Table-1

| Gene | SNP | Blocker | Cq numbers | | |
|------|-----|---------|---------|---------|-----|
| | | | NA18537 | NA18562 | ∆Cq |
| BRAF | rs3789806 G or C | - | 23.2 | 23.4 | 0.2 |
| | | C | 38.5 | 25.3 | 13.2 |
| | | G | 30.1 | 42.7 | 12.6 |
| EGF | rs11568849 C or A | - | 22.5 | 22.3 | 0.2 |
| | | A | 34.7 | 26.3 | 8.4 |
| | | C | 24.7 | 39.33 | 14.6 |
| IL23R | rs1884444 G or T | - | 22.2 | 22.4 | 0.2 |
| | | T | 36.1 | 24 | 12.1 |
| | | G | 28.3 | 35.4 | 7.1 |

(continued)

| Gene | SNP | Blocker | Cq numbers | | |
|------|-----|---------|-----------|---------|-----|
| | | | NA18537 | NA18562 | ΔCq |
| ALK | rs2246745 A or T | - | 22.6 | 22.3 | 0.3 |
| | | A | 23.2 | 33 | 9.8 |
| | | T | 33.2 | 24.1 | 9.1 |

Table-1 shows examples of allele specific amplification results for four set of SNP pairs in human genomic DNA samples NA18562 and NA18537, drawn from the 1000 Genomes database. For each SNP pair, a primer and blocker design for each allele variant were designed and tested. The base identities shown in the "Blocker" column indicates the allele that was being suppressed (variant), and "-" means no blocker was added. In all cases, large ΔCq values between the two alleles, ranging from 7.1 to 14.6 were observed. All the experiments were performed in triplicates using 400 nM of each primer and 4 μM of blocker in Bio-rad iTaq™ SYBR® Green Supermix. The performance of thermodynamically driven designs without any empirical optimization of primer and blocker sequences or experimental conditions were shown by the results.

Example 2

FIG. 5 shows an Example 2 of the present invention for the performance of a primer-blocker pair targeting human SNP rs3789806 (C/G) C allele at annealing/extension temperature ranging from 56°C to 66°C. The Example 2 showed good allele specific amplification from 56°C to 64°C and indicated at least 8°C of temperature robustness. 400 nM of each primer and 4 μM blocker were used, and other experimental conditions other than annealing/extension temperature were the same as described in Example 1.

Example 3

FIG. 6A and FIG. 6B show an Example 3 of the present invention. The designs of the Primers shown in the present invention provided more design space to avoid undesired interactions. The nucleic acid sequences of four sets of primer and blocker pairs that each specifically amplifies the human SNP rs3789806 C allele exhibited ΔCq > 11. 400 nM of each primer and 4 μM blocker were used, and other experimental conditions were the same as described in Example 1.

Example 4

FIG. 7A, FIG. 7B(I) and FIG. 7B(II) show an Example 4 of the present invention for controlling the allele-specific PCR behaviour via design of $\Delta G°_{rxn1}$. Simulations in FIG. 7A suggested that when the primer and blocker were designed so that $\Delta G°_{rxn1}$ was more positive, ΔCq values were greater. However, at more positive values of $\Delta G°_{rxn1}$, the amplification of the target was slowed and resulted in a larger value of Cq. Simulations were assumed as fixed $\Delta\Delta G°$ of 2 kcal/mol. The top sub graphs predicted the effects of the different values of $\Delta G°_{rxn1}$ (stars) and $\Delta G°_{rxn2}$ (dots) on the hybridization yields. The bottom sub graphs predicted simulated amplification curve of the target and the variant for each primer and blocker pair. In all cases depicted, the concentration ratio of blocker to primer was 20:1. FIG. 7B(I) and FIG. 7B(II) show experimental results of $\Delta G°_{rxn1}$ effects on amplification selectivity. Different $\Delta G°_{rxn1}$ values were achieved via lengthening or shortening the 3' end of the blocker. As expected, more positive $\Delta G°_{rxn1}$ resulted in larger Cq for both the target and the variant. An optimal intermediate $\Delta G°_{rxn1}$ resulted in largest ΔCq and relatively small Cq delay (Cq < 28) for the target. Unlike the simulations, ΔCq did not increase monotonically with $\Delta G°_{rxn1}$ because of the formation of primer-dimers and nonspecific amplification produced by a Cq ceiling. 200 nM of each primer and 2 μM blocker were used, and other experimental conditions were same as described in Example 1.

Example 5

FIG. 8 shows an Example 5 of the present invention for the blocker with a non-homologous sequence at the 3' end rather than a non-extensible chemical functionalization. Because the non-homologous sequences do not bind to the downstream region of either the target or the variant, the non-homologous region effectively prevents enzymatic extension. 400 nM of each primer and 4 μM blocker were used, and other experimental conditions were the same as described in Example 1. The target Cq and ΔCq values were similar to those shown in FIG. 4, indicating that like functional groups such as a 3-carbon spacer or a didexoynucleotide, a non-homologous sequence at the 3' end effectively prevents enzymatic extension. In FIG. 8, fP, rP, and B represent the forward primer, the reverse primer, and the blocker (for the forward primer), respectively.

Example 6

FIG. 9A and FIG. 9B show an Example 6 of the present invention. For a real-time PCR implementation of our non-allele-specific primer and allele-specific blocker, a reverse primer was also needed, which was not allele-specific. SYBR® Green Supermix and TaqMan® were used in the experiments and the results were not significantly different between the two. The Primer-blocker systems designed for human BRAF rs3789806 and IL23R rs 1884444 SNPs were multiplexed in the same reaction. TaqMan® probes targeting the corresponding downstream of the blocker binding regions were designed and used as the readout mechanism. The TaqMan® probe for BRAF rs3789806 amplicons was modified with a FAM fluorophore, an Iowa Black FQ quencher, and an internal ZEN quencher, and the TaqMan® probe for IL23R rs1884444 amplicons was modified with a Cy5 fluorophore and an Iowa Black RQ quencher. FIG. 9A shows the amplification results of multiplexed detection of BRAF rs3789806 G allele and IL23R rs1884444 T allele. The base identities denoted which allele the blockers were suppressing (variant). FIG. 9B shows multiplexed detection of BRAF rs3789806 C allele and IL23R rs1884444 G allele. In each experiment, the genomic DNA sample was mixed with 400 nM of each primer, 4 μM of each blocker, and 200 nM of each TaqMan® probe in Bio-rad iQ Supermix. The qPCR protocol was the same as indicated in Example 1.

Example 7

FIG. 10 shows an Example 7 of the present invention for nonspecific binding of primer or blocker to other primers, blockers, or templates. The Example 7 resulted in nonspecific amplification. The lower panel provided a design of a protector that suppressed the nonspecific binding of the primer and blocker. The blocker possessed a new region 11, whose sequence was non complementary to region 5 on the nucleic acid sequence, and the protector possessed a new region 10, whose sequence was complementary to region 11.

Example 8

FIG. 11A and FIG. 11B show an Example 8 of the present invention for primer and blocker designs for detecting a deletion and an insertion. Del rs200841330 was used to show proof-of-concept results. 400 nM of each primer and 4 μM blocker were used, and other experimental conditions were same as indicated in Example 1. Since the primer is non-allele-specific, this method can also be used to detect deletion or insertion with uncertain number of bases or uncertain position.

Example 9

FIG. 12 shows an Example 9 of the present invention by placing the primer to the 3' of the SNP position and blocker to the 5' of the primer. The Example 9 generated hybridization specificity only in the first cycle, so the ΔCq was smaller than the preferred design. The primer concentration was 400 nM, and the blocker concentration was 4 μM. Experimental conditions were the same as indicated in Example 1.

Example 10

FIG. 13 shows an Example 10 of the present invention for selective amplification of fungal 18S DNA subsequences of 3 pathogenic fungi species in large excess of homologous human DNA. Because fungal 18S subsequences and its homologous human subsequence differ in multiple regions, corresponding blocker species for both forward primer and reverse primer to maximize amplification specificity were designed. gBlock fragments (sequence verified 400bp double-stranded DNA, IDT) of 18S subsequences from 3 fungi species and the consensus sequence in human were used in the experiments. 60,000 copies (0.1%), 60 copies (0.0001%) and 0 copies (100% Human) of each fungal DNA gBlock fragment were separately mixed with 60,000,000 copies of human DNA. In all cases, results detected rare sequences down to 1 part in 1 million. 200 nM of each primer and 1 μM of each blocker were used, and other experimental conditions were the same as indicated in Example 1.

Example 11

FIG. 14 shows an Example 11 of the present invention. FIG. 14 describes the use of allele-specific amplification for both the forward and the reverse primer; two sets of variant-allele blockers, one for each primer were used. The blockers were partially complementary to each other, but at the operational conditions did not support predominant hybridization to one another. The blockers and primers were designed so that the forward primer cannot extend off the reverse blocker, and the reverse primer cannot extend off the forward blocker. FIG. 14 displayed preliminary experimental results.

The foregoing description of specific embodiments of the present disclosure has been presented for purpose of illustration and description. The exemplary embodiment was chosen and described in order to best explain the principles of the invention and its practical application, to thereby enable others skilled in the art to best utilize the invention.

**Claims**

1. A method for amplification of a target sequence comprising the steps of:

    (a) obtaining a sample containing one or more copies of a first nucleic acid comprising a variant sequence and possibly containing at least one copy of a second nucleic acid comprising the target sequence, wherein the target sequence and variant sequence each comprise a homologous subsequence and a variable subsequence, wherein the variable subsequence comprises at least one nucleotide, and wherein the variable subsequence of the target sequence is a target-specific subsequence and the variable subsequence of the variant sequence is a non-target specific subsequence;
    (b) introducing a blocker oligonucleotide to the sample comprising a first sequence comprising a target-neutral subsequence and a blocker variable subsequence, wherein the blocker variable subsequence is flanked on its 3' and 5' ends by the target-neutral subsequence and is continuous with the target-neutral subsequence, wherein the blocker variable subsequence divides the target-neutral subsequence into two portions, a portion to the 3' of the blocker variable subsequences and a portion to the 5' of the blocker variable subsequence, wherein the target-neutral subsequence is complementary to a portion of a homologous subsequence of both a first target nucleic acid and a first variant nucleic acid, wherein the blocker variable subsequence is complementary to a first variant subsequence of the first variant nucleic acid, and wherein the blocker oligonucleotide comprises a non-complementary sequence region at its 3' end, which prevents enzymatic extension and which is not complementary to a sequence continuous with the target-neutral subsequence; and
    (c) introducing a first primer oligonucleotide to the sample, wherein the first primer oligonucleotide is sufficient to induce enzymatic extension, wherein the first primer oligonucleotide comprises a second sequence, wherein the second sequence is complementary to a second portion of the homologous subsequence, wherein the second sequence overlaps the target-neutral subsequence of the blocker oligonucleotide by at least 5 nucleotides such that the second sequence comprises an overlapping subsequence and a non-overlapping subsequence, and wherein the second sequence does not include any sequence complementary to the variable subsequence; wherein the second sequence of the first primer oligonucleotide yields a standard free energy of hybridization ($\Delta G°PT$) to the target sequence, and the first sequence of the blocker oligonucleotide yields a standard free energy of hybridization ($\Delta G°_{BT}$) to the target sequence, which satisfies the following condition: $+2 \text{ kcal/mol} \geq \Delta G°_{PT} - \Delta G°_{BT} \geq -8 \text{ kcal/mol}$; and wherein the non-overlapping subsequence yields a standard free energy of hybridization ($\Delta G°3$) to the target sequence, which satisfies the following condition: $-4 \text{ kcal/mol} \geq \Delta G°_3 \geq -12 \text{ kcal/mol}$;
    (d) introducing to the sample a DNA polymerase, nucleoside triphosphates, and one or more reagents necessary for polymerase-based nucleic acid amplification; and
    (e) reacting the sample under conditions sufficient to achieve nucleic acid amplification.

2. The method of claim 1, wherein the DNA polymerase is a thermostable DNA polymerase, and wherein the conditions sufficient to achieve nucleic acid amplification comprise exposing the sample to at least 10 cycles, wherein each cycle comprises at least two different temperature exposures, one temperature exposure of at least 85°C, and one temperature exposure of no more than 75°C.

3. The method of claim 1, wherein the overlapping subsequence comprises a portion of the 5' end of the target-neutral subsequence, wherein said portion is from about 5 nucleotides to about 40 nucleotides, or optionally, is from about 7 nucleotides to about 30 nucleotides.

4. The method of claim 1, wherein the concentration of the blocker oligonucleotide introduced into the sample is about 2 to about 10,000 times greater than the concentration of the first primer oligonucleotide introduced into the sample.

5. The method of claim 1, wherein the concentration of the blocker oligonucleotide introduced into the sample is about 5 to about 1,000 times greater than the concentration of the first primer oligonucleotide introduced into the sample.

6. An oligonucleotide composition for carrying out the method of any of claims 1-5, comprising:

(a) a first blocker oligonucleotide comprising a first sequence comprising a first target-neutral subsequence and a first blocker variable subsequence, wherein the first blocker variable subsequence is flanked on its 3' and 5' ends by the first target-neutral subsequence and is continuous with the first target-neutral subsequence, wherein the first blocker variable subsequence divides the first target-neutral subsequence into two portions, a portion to the 3' of the first blocker variable subsequences and a portion to the 5' of the first blocker variable subsequence, wherein the first target-neutral subsequence is complementary to a first portion of a homologous subsequence of both a first target nucleic acid and a first variant nucleic acid, wherein the first blocker variable subsequence is complementary to a first variant subsequence of the first variant nucleic acid, and wherein the first blocker oligonucleotide comprises a non-complementary sequence region at its 3' end, which prevents enzymatic extension and which is not complementary to a sequence continuous with the first target-neutral subsequence; and
(b) a first primer oligonucleotide sufficient to induce enzymatic extension for amplification of a desired first target sequence, wherein the first primer oligonucleotide comprises a second sequence that is complementary to a second portion of a homologous subsequence of both the first target nucleic acid and the first variant nucleic acid, wherein the second sequence overlaps the target-neutral subsequence by at least 5 nucleotides such that the second sequence comprises a first overlapping subsequence and a first non-overlapping subsequence, and wherein the second sequence does not include the first blocker variable subsequence,

wherein the second sequence yields a standard free energy of hybridization ($\Delta G°PT$) to the target nucleic acid, and wherein the first sequence yields a standard free energy of hybridization ($\Delta G°BT$) to the target nucleic acid, which satisfies the following condition:

$$+2 \text{ kcal/mol} \geq \Delta G°_{PT} - \Delta G°_{BT} \geq -8 \text{ kcal/mol};$$

and
wherein the first non-overlapping subsequence of the first primer oligonucleotide yields a standard free energy of hybridization ($\Delta G°3$) to the target nucleic acid, which satisfies the following condition:

$$-4 \text{ kcal/mol} \geq \Delta G°_3 \geq -12 \text{ kcal/mol};$$

and

(c) a second blocker oligonucleotide comprising a fourth sequence comprising a second target-neutral subsequence and a second blocker variable subsequence, wherein the second blocker variable subsequence is flanked on its 3' and 5' ends by the second target-neutral subsequence and is continuous with the second target-neutral subsequence, wherein the second blocker variable subsequence divides the second target-neutral subsequence into two portions, a portion to the 3' of the second blocker variable subsequences and a portion to the 5' of the second blocker variable subsequence, wherein the second target-neutral subsequence is complementary to a first portion of a homologous subsequence of both a second target nucleic acid and a second variant nucleic acid, wherein the second blocker variable subsequence is complementary to a second variant subsequence of the second variant nucleic acid, and wherein the second blocker oligonucleotide comprises a non-complementary sequence region at its 3' end, which prevents enzymatic extension and which is not complementary to a sequence continuous with the second target-neutral subsequence; and
(d) a second primer oligonucleotide sufficient to induce enzymatic extension for amplification of a desired second target sequence, wherein the second primer oligonucleotide comprises a third sequence that is complementary to a second portion of a homologous subsequence of both the second target nucleic acid and the second variant nucleic acid, wherein the third sequence overlaps the second target-neutral subsequence by at least 5 nucleotides such that the third sequence comprises a second overlapping subsequence and a second non-overlapping subsequence, and wherein the third sequence does not include the second blocker variable subsequence,

wherein the third sequence yields a standard free energy of hybridization ($\Delta G°PT$) to the second target nucleic acid, and wherein the fourth sequence yields a standard free energy of hybridization ($\Delta G°BT$) to

the target nucleic acid, which satisfies the following condition:

$$+2 \text{ kcal/mol} \geq \Delta G°_{PT} - \Delta G°_{BT} \geq -8 \text{ kcal/mol};$$

and

wherein the second non-overlapping subsequence of the second primer oligonucleotide yields a standard free energy of hybridization ($\Delta G°3$) to the second target nucleic acid, which satisfies the following condition:

$$-4 \text{ kcal/mol} \geq \Delta G°_3 \geq -12 \text{ kcal/mol}.$$

**7.** The oligonucleotide composition of claim 6, wherein:

(a) the first overlapping subsequence comprises a portion of the 5' end of the first target-neutral subsequence, wherein the portion is from about 5 nucleotides to about 40 nucleotides;
(b) the second overlapping subsequence comprises a portion of the 5' end of the second target-neutral subsequence, wherein the portion is from about 5 nucleotides to about 40 nucleotides; or
(c) both (a) and (b).

**8.** The oligonucleotide composition of claim 6, wherein:

(a) the first overlapping subsequence comprises a portion of the 5' end of the first target-neutral subsequence, wherein the portion is from about 7 nucleotides to about 30 nucleotides;
(b) the second overlapping subsequence comprises a portion of the 5' end of the second target-neutral subsequence, wherein the portion is from about 7 nucleotides to about 30 nucleotides; or
(c) both (a) and (b).

**9.** The oligonucleotide composition of claim 6, wherein:

(a) the concentration of the first blocker oligonucleotide is about 2 to about 10,000 times greater than the concentration of the first primer oligonucleotide;
(b) the concentration of the second blocker oligonucleotide is about 2 to about 10,000 times greater than the concentration of the first primer oligonucleotide; or
(c) both (a) and (b).

**10.** The oligonucleotide composition of claim 6, wherein:

(a) the concentration of the first blocker oligonucleotide is about 5 to about 1,000 times greater than the concentration of the first primer oligonucleotide;
(b) the concentration of the second blocker oligonucleotide is about 5 to about 1,000 times greater than the concentration of the second primer oligonucleotide; or
(c) both (a) and (b).

**11.** The oligonucleotide composition of claim 6, wherein the oligonucleotide composition further comprises:

(a) a reagent necessary for polymerase chain reaction; or
(b) a plurality of nucleoside triphosphates; or
(c) a DNA polymerase.

**12.** The oligonucleotide composition of claim 6, wherein:

(a) the first blocker variable subsequence is a single nucleotide;
(b) the second blocker variable subsequence is a single nucleotide; or
(c) both (a) and (b).

**13.** The oligonucleotide composition of claim 6, wherein:

(a) the concentration of the first blocker oligonucleotide is about 10 to about 500 times greater than the concentration of the first primer oligonucleotide;

(b) the concentration of the second blocker oligonucleotide is about 10 to about 500 times greater than the concentration of the second primer oligonucleotide.

**14.** The oligonucleotide composition of claim 6, further comprising a target nucleic acid.

**Patentansprüche**

**1.** Ein Verfahren zur Amplifikation einer Zielsequenz, welches die folgenden Schritte umfasst:

(a) Erhalten einer Probe, die eine oder mehrere Kopien einer ersten Nukleinsäure enthält, die eine varianten Sequenz umfasst und möglicherweise mindestens eine Kopie einer zweiten Nukleinsäure enthält, die die Zielsequenz umfasst, wobei die Zielsequenz und die varianten Sequenz jeweils eine homologe Teilsequenz und eine variable Teilsequenz umfasst, wobei die variable Teilsequenz mindestens ein Nukleotid umfasst und wobei die variable Teilsequenz der Zielsequenz eine zielspezifische Teilsequenz ist und die variable Teilsequenz der varianten Sequenz eine nicht zielspezifische Teilsequenz ist;

(b) Einführen eines Blocker-Oligonukleotids in die Probe, umfassend eine erste Sequenz, umfassend eine zielneutrale Teilsequenz und eine variable Blocker-Teilsequenz, wobei die variable Blocker-Teilsequenz an ihren 3'- und 5'-Enden von der zielneutralen Teilsequenz flankiert wird und mit der zielneutralen Teilsequenz kontinuierlich ist, wobei die variable Blocker-Teilsequenz die zielneutrale Teilsequenz in zwei Abschnitte unterteilt, einen Abschnitt bis zum 3'-Ende der variablen Blocker-Teilsequenz und einen Abschnitt bis zum 5'-Ende der variablen Blocker-Teilsequenz, wobei die zielneutrale Teilsequenz komplementär zu einem Abschnitt einer homologen Teilsequenz sowohl einer ersten Zielnukleinsäure als auch einer ersten varianten Nukleinsäure ist, wobei die variable Blocker-Teilsequenz komplementär zu einer ersten varianten Teilsequenz der ersten varianten Nukleinsäure ist, und wobei das Blocker-Oligonukleotid an seinem 3'-Ende eine nicht-komplementäre Sequenzregion umfasst, die eine enzymatische Verlängerung verhindert und die nicht komplementär zu einer Sequenz ist, die mit der zielneutralen Teilsequenz kontinuierlich ist; und

(c) Einführen eines ersten Primer-Oligonukleotids in die Probe, wobei das erste Primer-Oligonukleotid ausreicht, um eine enzymatische Verlängerung zu induzieren, wobei das erste Primer-Oligonukleotid eine zweite Sequenz umfasst, wobei die zweite Sequenz zu einem zweiten Abschnitt der homologen Teilsequenz komplementär ist, wobei die zweite Sequenz die zielneutrale Teilsequenz des Blocker-Oligonukleotide um mindestens 5 Nukleotide überlappt, so dass die zweite Sequenz eine überlappende Teilsequenz und eine nicht überlappende Teilsequenz umfasst, und wobei die zweite Sequenz keine Sequenz umfasst, die zur variablen Teilsequenz komplementär ist; wobei die zweite Sequenz des ersten Primer-Oligonukleotids eine freie Standard-Hybridisierungsenergie ($\Delta G°_{PT}$) zur Zielsequenz ergibt und die erste Sequenz des Blocker-Oligonukleotids eine freie Standard-Hybridisierungsenergie ($\Delta G°_{BT}$) zur Zielsequenz ergibt, die die folgende Bedingung erfüllt: $+2$ kcal/mol $\geq \Delta G°_{PT} - \Delta G°_{BT} \geq -8$ kcal/mol; und wobei die nicht überlappende Teilsequenz eine freie Standardhybridisierungsenergie ($\Delta G°_3$) zur Zielsequenz ergibt, die die folgende Bedingung erfüllt: $-4$ kcal/mol $\geq \Delta G°_3 \geq -12$ kcal/mol;

(d) Zugabe zu der Probe einer DNA-Polymerase, von Nukleosidtriphosphaten und eines oder mehrerer Reagenzien, die für die Polymerase-basierte Nukleinsäureamplifikation erforderlich sind; und

(e) Umsetzen der Probe unter Bedingungen, die ausreichen, um eine Nukleinsäureamplifikation zu erreichen.

**2.** Das Verfahren nach Anspruch 1, wobei die DNA-Polymerase eine thermostabile DNA-Polymerase ist und wobei die Bedingungen, die ausreichen, um eine Nukleinsäureamplifikation zu erreichen, das Aussetzen der Probe um mindestens 10 Zyklen umfassen, wobei jeder Zyklus mindestens zwei verschiedene Temperatur-Expositionen, eine Temperatur-Exposition von mindestens 85 °C und eine Temperatur-Exposition von nicht mehr als 75 °C umfasst.

**3.** Das Verfahren nach Anspruch 1, wobei die überlappende Teilsequenz einen Abschnitt des 5'-Endes der zielneutralen Teilsequenz umfasst, wobei der Abschnitt etwa 5 Nukleotide bis etwa 40 Nukleotide oder gegebenenfalls etwa 7 Nukleotide bis etwa 30 Nukleotide beträgt.

**4.** Das Verfahren nach Anspruch 1, wobei die Konzentration des in die Probe eingebrachten Blocker-Oligonukleotids etwa 2- bis etwa 10.000-mal größer ist als die Konzentration des ersten Primer-Oligonukleotids, das in die Probe eingebracht wird.

**5.** Das Verfahren nach Anspruch 1, wobei die Konzentration des in die Probe eingebrachten Blocker-Oligonukleotids etwa 5- bis etwa 1.000-mal größer ist als die Konzentration des ersten Primer-Oligonukleotids, das in die Probe eingebracht wird.

6. Eine Oligonukleotidzusammensetzung zur Durchführung des Verfahrens nach einem der Ansprüche 1 bis 5, welche Folgendes umfasst:

(a) einen ersten Blocker-Oligonukleotid, umfassend eine erste Sequenz, umfassend eine erste zielneutrale Teilsequenz und eine erste variable Blocker-Teilsequenz, wobei die erste variable Blocker-Teilsequenz an ihren 3'- und 5'-Enden von der erste zielneutralen Teilsequenz flankiert wird und mit der erste zielneutralen Teilsequenz kontinuierlich ist, wobei die erste variable Blocker-Teilsequenz die erste zielneutrale Teilsequenz in zwei Abschnitte unterteilt, einen Abschnitt bis zum 3'-Ende der erste variablen Blocker-Teilsequenz und einen Abschnitt bis zum 5'-Ende der erste variablen Blocker-Teilsequenz, wobei die erste zielneutrale Teilsequenz komplementär zu einem ersten Abschnitt einer homologen Teilsequenz sowohl einer ersten Zielnukleinsäure als auch einer ersten varianten Nukleinsäure ist, wobei die erste variable Blocker-Teilsequenz komplementär zu einer ersten varianten Teilsequenz der ersten varianten Nukleinsäure ist, und wobei das erste Blocker-Oligonukleotid an seinem 3'-Ende eine nicht-komplementäre Sequenzregion umfasst, die eine enzymatische Verlängerung verhindert und die nicht komplementär zu einer Sequenz ist, die mit der ersten zielneutralen Teilsequenz kontinuierlich ist; und

(b) ein erstes Primer-Oligonukleotid, das ausreicht, um eine enzymatische Verlängerung zur Amplifikation einer gewünschten ersten Zielsequenz zu induzieren, wobei das erste Primer-Oligonukleotid eine zweite Sequenz umfasst, die zu einem zweiten Abschnitt einer homologen Teilsequenz sowohl der ersten Zielnukleinsäure als auch der ersten varianten Nukleinsäure komplementär ist, wobei die zweite Sequenz die zielneutrale Teilsequenz um mindestens 5 Nukleotide überlappt, so dass die zweite Sequenz eine erste überlappende Teilsequenz und eine erste nicht überlappende Teilsequenz umfasst, und wobei die zweite Sequenz nicht die erste variable Blocker-Teilsequenz umfasst,

wobei die zweite Sequenz eine freie Standard-Hybridisierungsenergie ($\Delta G°PT$) für die Zielnukleinsäure ergibt und wobei die erste Sequenz eine freie Standard-Hybridisierungsenergie ($\Delta G°BT$) für die Zielnukleinsäure ergibt, die die folgende Bedingung erfüllt:

$$+2 \ \text{kcal/mol} \geq \Delta G°_{PT} - \Delta G°_{BT} \geq -8 \ \text{kcal/mol};$$

und

wobei die erste nicht überlappende Teilsequenz des ersten Primer-Oligonukleotids eine freie Standard-Hybridisierungsenergie ($\Delta G°3$) zur Zielnukleinsäure ergibt, die die folgende Bedingung erfüllt:

$$-4 \ \text{kcal/mol} \geq \Delta G°_{3} \geq -12 \ \text{kcal/mol};$$

und

(c) einen zweiten Blocker-Oligonukleotid, umfassend eine vierte Sequenz, umfassend eine zweite zielneutrale Teilsequenz und eine zweite variable Blocker-Teilsequenz, wobei die zweite variable Blocker-Teilsequenz an ihren 3'- und 5'-Enden von der zweite zielneutralen Teilsequenz flankiert wird und mit der zweite zielneutralen Teilsequenz kontinuierlich ist, wobei die zweite variable Blocker-Teilsequenz die zweite zielneutrale Teilsequenz in zwei Abschnitte unterteilt, einen Abschnitt bis zum 3'-Ende der zweite variablen Blocker-Teilsequenz und einen Abschnitt bis zum 5'-Ende der zweite variablen Blocker-Teilsequenz, wobei die zweite zielneutrale Teilsequenz komplementär zu einem ersten Abschnitt einer homologen Teilsequenz sowohl einer zweiten Zielnukleinsäure als auch einer zweiten varianten Nukleinsäure ist, wobei die zweite variable Blocker-Teilsequenz komplementär zu einer zweiten varianten Teilsequenz der zweiten varianten Nukleinsäure ist, und wobei das zweite Blocker-Oligonukleotid an seinem 3'-Ende eine nicht-komplementäre Sequenzregion umfasst, die eine enzymatische Verlängerung verhindert und die nicht komplementär zu einer Sequenz ist, die mit der zweiten zielneutralen Teilsequenz kontinuierlich ist; und

(d) ein zweites Primer-Oligonukleotid, das ausreicht, um eine enzymatische Verlängerung zur Amplifikation einer gewünschten zweiten Zielsequenz zu induzieren, wobei das zweite Primer-Oligonukleotid eine dritte Sequenz umfasst, die zu einem zweiten Abschnitt einer homologen Teilsequenz sowohl der zweiten Zielnukleinsäure als auch der zweiten varianten Nukleinsäure komplementär ist, wobei die dritte Sequenz die zweite zielneutrale Teilsequenz um mindestens 5 Nukleotide überlappt, so dass die dritte Sequenz eine zweite überlappende Teilsequenz und eine zweite nicht überlappende Teilsequenz umfasst, und wobei die dritte Sequenz nicht die zweite variable Blocker-Teilsequenz umfasst,

wobei die dritte Sequenz eine freie Standard-Hybridisierungsenergie ($\Delta$G°PT) für die zweite Zielnukleinsäure ergibt und wobei die vierte Sequenz eine freie Standard-Hybridisierungsenergie ($\Delta$G°BT) für die Zielnukleinsäure ergibt, die die folgende Bedingung erfüllt:

$$+2 \text{ kcal/mol} \geq \Delta G^\circ_{PT} - \Delta G^\circ_{BT} \geq -8 \text{ kcal/mol};$$

und

wobei die zweite nicht überlappende Teilsequenz des zweiten Primer-Oligonukleotids eine freie Standard-Hybridisierungsenergie ($\Delta$G°3) zur zweiten Zielnukleinsäure ergibt, die die folgende Bedingung erfüllt:

$$-4 \text{ kcal/mol} \geq \Delta G^\circ_3 \geq -12 \text{ kcal/mol}.$$

**7.** Die Oligonukleotid-Zusammensetzung nach Anspruch 6, wobei:

(a) die erste überlappende Teilsequenz einen Abschnitt des 5'-Endes der ersten zielneutralen Teilsequenz umfasst, wobei der Abschnitt etwa 5 bis etwa 40 Nukleotide beträgt;
(b) die zweite überlappende Teilsequenz einen Abschnitt des 5'-Endes der zweiten zielneutralen Teilsequenz umfasst, wobei der Abschnitt etwa 5 Nukleotide bis etwa 40 Nukleotide beträgt; oder
(c) sowohl (a) als auch (b).

**8.** Die Oligonukleotid-Zusammensetzung nach Anspruch 6, wobei:

(a) die erste überlappende Teilsequenz einen Abschnitt des 5'-Endes der ersten zielneutralen Teilsequenz umfasst, wobei der Abschnitt etwa 7 bis etwa 30 Nukleotide beträgt;
(b) die zweite überlappende Teilsequenz einen Abschnitt des 5'-Endes der zweiten zielneutralen Teilsequenz umfasst, wobei der Abschnitt etwa 7 Nukleotide bis etwa 30 Nukleotide beträgt; oder
(c) sowohl (a) als auch (b).

**9.** Die Oligonukleotid-Zusammensetzung nach Anspruch 6, wobei:

(a) die Konzentration des ersten Blocker-Oligonukleotids etwa 2- bis etwa 10.000-mal größer ist als die Konzentration des ersten Primer-Oligonukleotids;
(b) die Konzentration des zweiten Blocker-Oligonukleotids etwa 2- bis etwa 10.000-mal größer ist als die Konzentration des ersten Primer-Oligonukleotids; oder
(c) sowohl (a) als auch (b).

**10.** Die Oligonukleotid-Zusammensetzung nach Anspruch 6, wobei:

(a) die Konzentration des ersten Blocker-Oligonukleotids etwa 5- bis etwa 1.000-mal größer ist als die Konzentration des ersten Primer-Oligonukleotids;
(b) die Konzentration des zweiten Blocker-Oligonukleotids etwa 5- bis etwa 1.000-mal größer ist als die Konzentration des zweiten Primer-Oligonukleotids; oder
(c) sowohl (a) als auch (b).

**11.** Die Oligonukleotid-Zusammensetzung nach Anspruch 6, wobei die Oligonukleotid-Zusammensetzung ferner Folgendes umfasst:

(a) ein für die Polymerase-Kettenreaktion erforderliches Reagens; oder
(b) eine Vielzahl von Nukleosidtriphosphaten; oder
(c) eine DNA-Polymerase.

**12.** Die Oligonukleotid-Zusammensetzung nach Anspruch 6, wobei:

(a) die erste variable Blocker-Teilsequenz ein einzelnes Nukleotid ist;
(b) die zweite variable Blocker-Teilsequenz ein einzelnes Nukleotid ist; oder
(c) sowohl (a) als auch (b).

**13.** Die Oligonukleotid-Zusammensetzung nach Anspruch 6, wobei:

(a) die Konzentration des ersten Blocker-Oligonukleotids etwa 10- bis etwa 500-mal größer ist als die Konzentration des ersten Primer-Oligonukleotids;
(b) die Konzentration des zweiten Blocker-Oligonukleotids etwa 10- bis etwa 500-mal größer ist als die Konzentration des zweiten Primer-Oligonukleotids.

**14.** Die Oligonukleotid-Zusammensetzung nach Anspruch 6, die ferner eine Zielnukleinsäure umfasst.

## Revendications

**1.** Procédé d'amplification d'une séquence cible comprenant les étapes suivantes :

(a) l'obtention d'un échantillon contenant une ou plusieurs copies d'un premier acide nucléique comprenant une séquence variante et contenant éventuellement au moins une copie d'un second acide nucléique comprenant la séquence cible, dans lequel la séquence cible et la séquence variante comprennent chacune une sous-séquence homologue et une sous-séquence variable, dans lequel la sous-séquence variable comprend au moins un nucléotide, et dans lequel la sous-séquence variable de la séquence cible est une sous-séquence spécifique à la cible et la sous-séquence variable de la séquence variante est une sous-séquence non spécifique à la cible ;
(b) l'introduction d'un oligonucléotide bloqueur dans l'échantillon comprenant une première séquence comprenant une sous-séquence neutre vis-à-vis de la cible et une sous-séquence variable de bloqueur, dans lequel la sous-séquence variable de bloqueur est flanquée sur ses extrémités 3' et 5' par la sous-séquence neutre vis-à-vis de la cible et est continue avec la sous-séquence neutre vis-à-vis de la cible, dans lequel la sous-séquence variable de bloqueur divise la sous-séquence neutre vis-à-vis de la cible en deux parties, une partie en 3' des sous-séquences variables de bloqueurs et une partie en 5' de la sous-séquence variable de bloqueur, dans lequel la sous-séquence neutre vis-à-vis de la cible est complémentaire d'une partie d'une sous-séquence homologue à la fois d'un premier acide nucléique cible et d'un premier acide nucléique variant, dans lequel la sous-séquence variable de bloqueur est complémentaire d'une première sous-séquence variante du premier acide nucléique variant, et dans lequel l'oligonucléotide bloqueur comprend une région de séquence non complémentaire à son extrémité 3', qui empêche l'extension enzymatique et qui n'est pas complémentaire d'une séquence continue avec la sous-séquence neutre vis-à-vis de la cible ; et
(c) l'introduction d'un premier oligonucléotide d'amorce dans l'échantillon, dans lequel le premier oligonucléotide d'amorce est suffisant pour induire une extension enzymatique, dans lequel le premier oligonucléotide d'amorce comprend une seconde séquence, dans lequel la seconde séquence est complémentaire d'une seconde partie de la sous-séquence homologue, dans lequel la seconde séquence chevauche la sous-séquence neutre vis-à-vis de la cible de l'oligonucléotide bloqueur sur au moins 5 nucléotides de telle sorte que la seconde séquence comprend une sous-séquence chevauchante et une sous-séquence non chevauchante, et dans lequel la seconde séquence ne comprend aucune séquence complémentaire de la sous-séquence variable ; dans lequel la seconde séquence du premier oligonucléotide d'amorce donne une énergie libre standard d'hybridation ($\Delta G°_{PT}$) à la séquence cible, et la première séquence de l'oligonucléotide bloqueur donne une énergie libre standard d'hybridation ($\Delta G°_{BT}$) à la séquence cible, qui satisfait à la condition suivante : +2 kcal/mol $\geq \Delta G°_{PT}$ - $\Delta G°_{BT} \geq$ -8 kcal/mol ; et dans lequel la sous-séquence non chevauchante donne une énergie libre standard d'hybridation ($\Delta G°_3$) à la séquence cible, qui satisfait à la condition suivante : -4 kcal/mol $\geq \Delta G°_3 \geq$ -12 kcal/mol ;
(d) l'introduction dans l'échantillon d'une ADN polymérase, de nucléoside-triphosphates et d'un ou de plusieurs réactifs nécessaires pour une amplification d'acide nucléique par polymérase ; et
(e) la réaction de l'échantillon dans des conditions suffisantes pour obtenir une amplification d'acide nucléique.

**2.** Procédé selon la revendication 1, dans lequel l'ADN polymérase est une ADN polymérase thermostable, et dans lequel les conditions suffisantes pour obtenir une amplification d'acide nucléique comprennent l'exposition de l'échantillon à au moins 10 cycles, dans lequel chaque cycle comprend au moins deux expositions à des températures différentes, une exposition à une température d'au moins 85 °C, et une exposition à une température ne dépassant pas 75 °C.

**3.** Procédé selon la revendication 1, dans lequel la sous-séquence chevauchante comprend une partie de l'extrémité 5' de la sous-séquence neutre vis-à-vis de la cible, dans lequel ladite partie est d'environ 5 nucléotides à environ 40 nucléotides, ou facultativement, est d'environ 7 nucléotides à environ 30 nucléotides.

**4.** Procédé selon la revendication 1, dans lequel la concentration de l'oligonucléotide bloqueur introduit dans l'échantillon est environ 2 à environ 10 000 fois supérieure à la concentration du premier oligonucléotide d'amorce introduit dans l'échantillon.

**5.** Procédé selon la revendication 1, dans lequel la concentration de l'oligonucléotide bloqueur introduit dans l'échantillon est environ 5 à environ 1000 fois supérieure à la concentration du premier oligonucléotide d'amorce introduit dans l'échantillon.

**6.** Composition d'oligonucléotides pour mettre en œuvre le procédé selon l'une quelconque des revendications 1 à 5, comprenant :

(a) un premier oligonucléotide bloqueur comprenant une première séquence comprenant une première sous-séquence neutre vis-à-vis de la cible et une première sous-séquence variable de bloqueur, dans laquelle la première sous-séquence variable de bloqueur est flanquée sur ses extrémités 3' et 5' par la première sous-séquence neutre vis-à-vis de la cible et est continue avec la première sous-séquence neutre vis-à-vis de la cible, dans laquelle la première sous-séquence variable de bloqueur divise la première sous-séquence neutre vis-à-vis de la cible en deux parties, une partie en 3' des premières sous-séquences variables de bloqueur et une partie en 5' de la première sous-séquence variable de bloqueur, dans laquelle la première sous-séquence neutre vis-à-vis de la cible est complémentaire d'une première partie d'une sous-séquence homologue à la fois d'un premier acide nucléique cible et d'un premier acide nucléique variant, dans laquelle la première sous-séquence variable de bloqueur est complémentaire d'une première sous-séquence variante du premier acide nucléique variant, et dans laquelle le premier oligonucléotide bloqueur comprend une région de séquence non complémentaire à son extrémité 3', qui empêche l'extension enzymatique et qui n'est pas complémentaire d'une séquence continue avec la première sous-séquence neutre vis-à-vis de la cible ; et

(b) un premier oligonucléotide d'amorce suffisant pour induire une extension enzymatique pour l'amplification d'une première séquence cible souhaitée, dans laquelle le premier oligonucléotide d'amorce comprend une seconde séquence qui est complémentaire d'une seconde partie d'une sous-séquence homologue à la fois du premier acide nucléique cible et du premier acide nucléique variant, dans laquelle la seconde séquence chevauche la sous-séquence neutre vis-à-vis de la cible sur au moins 5 nucléotides de telle sorte que la seconde séquence comprend une première sous-séquence chevauchante et une première sous-séquence non chevauchante, et dans laquelle la seconde séquence ne comprend pas la première sous-séquence variable de bloqueur,

dans laquelle la seconde séquence donne une énergie libre standard d'hybridation ($\Delta$G°PT) à l'acide nucléique cible, et dans laquelle la première séquence donne une énergie libre standard d'hybridation ($\Delta$G°BT) à l'acide nucléique cible, qui satisfait à la condition suivante :

$$+2 \text{ kcal/mol} \geq \Delta G°_{PT} - \Delta G°_{BT} \geq -8 \text{ kcal/mol} \text{ ;}$$

et

dans laquelle la première sous-séquence non chevauchante du premier oligonucléotide d'amorce donne une énergie libre standard d'hybridation ($\Delta$G°3) à l'acide nucléique cible, qui satisfait à la condition suivante :

$$-4 \text{ kcal/mol} \geq \Delta G°_3 \geq -12 \text{ kcal/mol} \text{ ;}$$

et

(c) un second oligonucléotide bloqueur comprenant une quatrième séquence comprenant une seconde sous-séquence neutre vis-à-vis de la cible et une seconde sous-séquence variable de bloqueur, dans laquelle la seconde sous-séquence variable de bloqueur est flanquée sur ses extrémités 3' et 5' par la seconde sous-séquence neutre vis-à-vis de la cible et est continue avec la seconde sous-séquence neutre vis-à-vis de la cible, dans laquelle la seconde sous-séquence variable de bloqueur divise la seconde sous-séquence neutre vis-à-vis de la cible en deux parties, une partie en 3' des secondes sous-séquences variables de bloqueurs et une partie en 5' de la seconde sous-séquence variable de bloqueur, dans laquelle la seconde sous-séquence neutre vis-à-vis de la cible est complémentaire d'une première partie d'une sous-séquence homologue à la fois d'un second acide nucléique cible et d'un second acide nucléique variant, dans laquelle la seconde sous-séquence variable de bloqueur est complémentaire d'une seconde sous-séquence variante du second acide nucléique variant, et dans laquelle le second oligonucléotide bloqueur comprend une région de séquence non complémentaire à son extrémité 3', qui empêche l'extension enzymatique et qui n'est pas complémentaire d'une séquence continue avec la seconde sous-séquence neutre vis-à-vis de la cible ; et

(d) un second oligonucléotide d'amorce suffisant pour induire une extension enzymatique pour l'amplification d'une seconde séquence cible souhaitée, dans laquelle le second oligonucléotide d'amorce comprend une troisième séquence qui est complémentaire d'une seconde partie d'une sous-séquence homologue à la fois du second acide nucléique cible et du second acide nucléique variant, dans laquelle la troisième séquence chevauche la seconde sous-séquence neutre vis-à-vis de la cible sur au moins 5 nucléotides de telle sorte que la troisième séquence comprend une seconde sous-séquence chevauchante et une seconde sous-séquence non chevauchante, et dans laquelle la troisième séquence ne comprend pas la seconde sous-séquence variable de bloqueur,

dans laquelle la troisième séquence donne une énergie libre standard d'hybridation ($\Delta G°PT$) au second acide nucléique cible, et dans laquelle la quatrième séquence donne une énergie libre standard d'hybridation ($\Delta G°BT$) à l'acide nucléique cible, qui satisfait à la condition suivante :

$$+2 \ \text{kcal/mol} \geq \Delta G°_{PT} - \Delta G°_{BT} \geq -8 \ \text{kcal/mol} \ ;$$

et

dans laquelle la seconde sous-séquence non chevauchante du second oligonucléotide d'amorce donne une énergie libre standard d'hybridation ($\Delta G°3$) au second acide nucléique cible, qui satisfait à la condition suivante :

$$-4 \ \text{kcal/mol} \geq \Delta G°_{3} \geq -12 \ \text{kcal/mol.}$$

7. Composition d'oligonucléotides selon la revendication 6, dans laquelle :

(a) la première sous-séquence chevauchante comprend une partie de l'extrémité 5' de la première sous-séquence neutre vis-à-vis de la cible, dans laquelle la partie est d'environ 5 nucléotides à environ 40 nucléotides ;
(b) la seconde sous-séquence chevauchante comprend une partie de l'extrémité 5' de la seconde sous-séquence neutre vis-à-vis de la cible, dans laquelle la partie est d'environ 5 nucléotides à environ 40 nucléotides ; ou
(c) à la fois (a) et (b).

8. Composition d'oligonucléotides selon la revendication 6, dans laquelle :

(a) la première sous-séquence chevauchante comprend une partie de l'extrémité 5' de la première sous-séquence neutre vis-à-vis de la cible, dans laquelle la partie est d'environ 7 nucléotides à environ 30 nucléotides ;
(b) la seconde sous-séquence chevauchante comprend une partie de l'extrémité 5' de la seconde sous-séquence neutre vis-à-vis de la cible, dans laquelle la partie est d'environ 7 nucléotides à environ 30 nucléotides ; ou
(c) à la fois (a) et (b).

9. Composition d'oligonucléotides selon la revendication 6, dans laquelle :

(a) la concentration du premier oligonucléotide bloqueur est environ 2 à environ 10 000 fois supérieure à la concentration du premier oligonucléotide d'amorce ;
(b) la concentration du second oligonucléotide bloqueur est environ 2 à environ 10 000 fois supérieure à la concentration du premier oligonucléotide d'amorce ; ou
(c) à la fois (a) et (b).

10. Composition d'oligonucléotides selon la revendication 6, dans laquelle :

(a) la concentration du premier oligonucléotide bloqueur est environ 5 à environ 1000 fois supérieure à la concentration du premier oligonucléotide d'amorce ;
(b) la concentration du second oligonucléotide bloqueur est environ 5 à environ 1000 fois supérieure à la concentration du second oligonucléotide d'amorce ; ou
(c) à la fois (a) et (b).

11. Composition d'oligonucléotides selon la revendication 6, dans laquelle la composition d'oligonucléotides comprend en outre :

(a) un réactif nécessaire à une réaction en chaîne par polymérase ; ou
(b) une pluralité de nucléoside-triphosphates ; ou
(c) une ADN polymérase.

12. Composition d'oligonucléotides selon la revendication 6, dans laquelle :

(a) la première sous-séquence variable de bloqueur est un seul nucléotide ;
(b) la seconde sous-séquence variable de bloqueur est un seul nucléotide ; ou
(c) à la fois (a) et (b).

13. Composition d'oligonucléotides selon la revendication 6, dans laquelle :

(a) la concentration du premier oligonucléotide bloqueur est environ 10 à environ 500 fois supérieure à la concentration du premier oligonucléotide d'amorce ;
(b) la concentration du second oligonucléotide bloqueur est environ 10 à environ 500 fois supérieure à la concentration du second oligonucléotide d'amorce.

14. Composition d'oligonucléotides selon la revendication 6, comprenant en outre un acide nucléique cible.

FIG. 1

FIG. 2

Primer $\underbrace{1}\quad\underbrace{2}$
$\overline{\text{AGCTGACCTAA}}$

$\overline{\text{TCGACTGGATTC}\textbf{G}\text{CTA}}$
$\underbrace{5}\quad\underbrace{6}\quad\underbrace{7}$ Variant

Primer $\underbrace{1}\quad\underbrace{2}$
$\overline{\text{AGCTGACCTAA}}$

$\overline{\text{TCGACTGGATTC}\textbf{A}\text{CTA}}$
$\underbrace{5}\quad\underbrace{6}\quad\underbrace{8}$ Target

$\underbrace{3}\quad\underbrace{4}$ Blocker
$\overline{\text{ACCTAAG}\textbf{C}\text{GAT}}$

$\overline{\text{TCGACTGGATTC}\textbf{G}\text{CTA}}$
$\underbrace{5}\quad\underbrace{6}\quad\underbrace{7}$ Variant

$\underbrace{3}\quad\underbrace{4}$ Blocker
$\overline{\text{ACCTAAG}\textbf{C}\text{GAT}}$

$\overline{\text{TCGACTGGATTC}\textbf{A}\text{CTA}}$
$\underbrace{5}\quad\underbrace{6}\quad\underbrace{8}$ Target

$$\Delta G^{\circ}_{PV} = \Delta G^{\circ}_{1\text{-}5} + \Delta G^{\circ}_{2\text{-}6}$$

$$\Delta G^{\circ}_{1\text{-}5} = \Delta G^{\circ}_{init} + \Delta G^{\circ}\left(\tfrac{AG}{TC}\right) + \Delta G^{\circ}\left(\tfrac{GC}{CG}\right) + \Delta G^{\circ}\left(\tfrac{CT}{GA}\right) + \Delta G^{\circ}\left(\tfrac{TG}{AC}\right) + \Delta G^{\circ}\left(\tfrac{GA}{CT}\right)$$

$$\Delta G^{\circ}_{2\text{-}6} = \Delta G^{\circ}\left(\tfrac{AC}{TG}\right) + \Delta G^{\circ}\left(\tfrac{CC}{GG}\right) + \Delta G^{\circ}\left(\tfrac{CT}{GA}\right) + \Delta G^{\circ}\left(\tfrac{TA}{AT}\right) + \Delta G^{\circ}\left(\tfrac{AA}{TT}\right)$$

$$\Delta G^{\circ}_{PT} = \Delta G^{\circ}_{1\text{-}5} + \Delta G^{\circ}_{2\text{-}6} = \Delta G^{\circ}_{PV}$$

$$\Delta G^{\circ}_{BV} = \Delta G^{\circ}_{3\text{-}6} + \Delta G^{\circ}_{4\text{-}7}$$

$$\Delta G^{\circ}_{3\text{-}6} = \Delta G^{\circ}\left(\tfrac{AC}{TG}\right) + \Delta G^{\circ}\left(\tfrac{CC}{GG}\right) + \Delta G^{\circ}\left(\tfrac{CT}{GA}\right) + \Delta G^{\circ}\left(\tfrac{TA}{AT}\right) + \Delta G^{\circ}\left(\tfrac{AA}{TT}\right)$$

$$\Delta G^{\circ}_{4\text{-}7} = \Delta G^{\circ}_{init} + \Delta G^{\circ}\left(\tfrac{AG}{TC}\right) + \Delta G^{\circ}\left(\tfrac{GC}{CG}\right) + \Delta G^{\circ}\left(\tfrac{CG}{GC}\right) + \Delta G^{\circ}\left(\tfrac{GA}{CT}\right) + \Delta G^{\circ}\left(\tfrac{AT}{TA}\right)$$

$$\Delta G^{\circ}_{BT} = \Delta G^{\circ}_{3\text{-}6} + \Delta G^{\circ}_{4\text{-}8}$$

$$\Delta G^{\circ}_{4\text{-}8} = \Delta G^{\circ}_{init} + \Delta G^{\circ}\left(\tfrac{AG}{TC}\right) + \Delta G^{\circ}\left(\tfrac{GC}{CA}\right) + \Delta G^{\circ}\left(\tfrac{CG}{AC}\right) + \Delta G^{\circ}\left(\tfrac{GA}{CT}\right) + \Delta G^{\circ}\left(\tfrac{AT}{TA}\right)$$

FIG. 3

Forward Primer
CAGCCTCATCCAAAAGAGGAAA
$\Delta G^\circ_{rxn1}$ = 3.38 kcal/mol

Blocker AAAAGAGGAAA C AGGACCCCAGAGCTC

Reverse Primer Sequence

. . . . . AGTGTCGGAGTAGGTTTTCTCCTTT A TCCTGGGGTCTCGAGGGA . . . . . . TTACGACCCCAAATCTCACTC . . . . .
 Target NA18537

. . . . . AGTGTCGGAGTAGGTTTTCTCCTTT G TCCTGGGGTCTCGAGGGA . . . . . . TTACGACCCCAAATCTCACTC . . . . .
 Variant NA18562

SMAD7 rs4939827

100% Target
Cq = 24.1

0.1% Target
Cq = 34.7

100% WT
Cq = 38.9
ΔCq = 14.8

FIG. 4

| Extension Temperature (°C) | Target Cq | ΔCq |
|---|---|---|
| 56 | 28.4 | 12.5 |
| 58 | 24.7 | 8.9 |
| 60 | 25.3 | 13.2 |
| 62 | 26.7 | 13.5 |
| 64 | 36.8 | 17.0 |
| 66 | 58.7 | - |

Working Range: 56-64°C

Primer
TGTATATAGACGGTAAAATAAACACCAAGA

Blocker
ACACCAAGA^CGTGGTAAATATTTACCTGGTC

. . . . . AACATATATCTGCCATTTTATTTGTGGTTCTCCACCATTTATAAATGGACCAGGGAC . . . . . .
Target                                                                    NA18562

. . . . . AACATATATCTGCCATTTTATTTGTGGTTCTGCACCATTTATAAATGGACCAGGGAC . . . . . .
Variant                                                                   NA18537

BRAF rs3789806

FIG. 5

30

FIG. 6A

| Target Cq | ΔCq |
|-----------|------|
| 25.3 | 13.2 |
| 26.9 | 11.3 |
| 24.3 | 14.0 |
| 26.5 | 17.2 |

Primer-Blocker Pair 1

TGTATATAGACGGTAAAATAAACACCAAGA

ACACCAAGA^C GTGGTAAATATTTACCTGGTC

Primer-Blocker Pair 2

GAGCCTTGTATATAGACGGTAAAATAAAC

CGGTAAAATAAACACCAAGA^C GTGGTAAATATTTAC

Primer-Blocker Pair 3

TGGAGCCTTGTATATAGACGGTAAA

GACGGTAAAATAAACACCAAGA^C GTGGTAAA

Primer-Blocker Pair 4

ACTGGAGCCTTGTATATAGACGG

GTATATAGACGGTAAAATAAACACCAAGA^C GTGGT

......TTGACCTCGGAACATATATCTGCCATTTTATTTGTGGTTCT|C|CACCATTTATAAATGGACCAGGGAC......
Target

......TTGACCTCGGAACATATATCTGCCATTTTATTTGTGGTTCT|G|CACCATTTATAAATGGACCAGGGAC......
Variant

BRAF rs3789806

FIG. 6B

FIG. 7A

Experimental Validation on SMAD7 rs4939827

Primer

CAGCCTCATCCAAAAGAGGAAA

Blocker C1   AAAAGAGGAAA<sup>C</sup>AGGACCCCAGA

Blocker C2   AAAAGAGGAAA<sup>C</sup>AGGACCCCAGAG

Blocker C3   AAAAGAGGAAA<sup>C</sup>AGGACCCCAGAGC

Blocker C4   AAAAGAGGAAA<sup>C</sup>AGGACCCCAGAGCT

Blocker C5   AAAAGAGGAAA<sup>C</sup>AGGACCCCAGAGCTC

Blocker C6   AAAAGAGGAAA<sup>C</sup>AGGACCCCAGAGCTCC

| $\Delta G^{\circ}_{rxn1}$ (kcal/mol) | Target Cq | $\Delta$Cq |
|---|---|---|
| 0.13 | 23.2 | 6.1 |
| 0.73 | 23.3 | 10.9 |
| 2.19 | 23.6 | 12.9 |
| 2.78 | 23.7 | 15.4 |
| 3.38 | 24.8 | 17.4 |
| 4.54 | 25.6 | 14.7 |

. . . . . .AGTGTCGGAGTAGGTTTTCTCCTTT A TCCTGGGGTCTCGAGGGA . . . . . .
            Target

. . . . . .AGTGTCGGAGTAGGTTTTCTCCTTT G TCCTGGGGTCTCGAGGGA . . . . . .
            Variant

FIG. 7B(I)

Experimental Validation on SMAD7 rs4939827

Primer
CAGCCTCATCCAAAAGAGGAAA

Blocker T1  AAAAGAGGAAA(T)AGGACCCCAGA

Blocker T2  AAAAGAGGAAA(T)AGGACCCCAGAG

Blocker T3  AAAAGAGGAAA(T)AGGACCCCAGAGC

Blocker T4  AAAAGAGGAAA(T)AGGACCCCAGAGCT

Blocker T5  AAAAGAGGAAA(T)AGGACCCCAGAGCTC

Blocker T6  AAAAGAGGAAA(T)AGGACCCCAGAGCTCC

| $\Delta G^{\circ}_{rxn1}$ (kcal/mol) | Target Cq | $\Delta$Cq |
|---|---|---|
| -2.16 | 23.3 | 1.7 |
| -1.57 | 23.5 | 6.6 |
| -0.11 | 25.8 | 10.1 |
| 0.49 | 27.3 | 9.3 |
| 1.08 | 29.9 | 8.8 |
| 2.24 | 32.6 | 8.8 |

. . . . .AGTGTCGGAGTAGGTTTTCTCCTTT[G]TCCTGGGGTCTCGAGGGA . . . . . .
Target

. . . . .AGTGTCGGAGTAGGTTTTCTCCTTT[A]TCCTGGGGTCTCGAGGGA . . . . . .
Variant

FIG. 7B(II)

Primer

TGTATATAGACGGTAAAATAAACACCAAGA

Blocker ACACCAAGACGTGGTAAATATTTACCTGGTC AAAA

. . . . . AACATATATCTGCCATTTTATTTGTGGTTCTCCACCATTTATAAATGGACCAGGGAC . . . . .
Target

. . . . . AACATATATCTGCCATTTTATTTGTGGTTCTGCACCATTTATAAATGGACCAGGGAC . . . . .
Variant

BRAF rs3789806

FIG. 8

**FIG. 9A**

FAM Channel BRAF rs3789806 (C/G)

Cy5 Channel IL23R rs1884444 (G/T)

Blockers: BRAF-C + IL23R-G

— NA18537
- - - NA18562

**FIG. 9B**

FAM Channel BRAF rs3789806 (C/G)

Cy5 Channel IL23R rs1884444 (G/T)

Blockers: BRAF-G + IL23R-T

Possible Nonspecific Binding of Primer and Blocker to Other Templates in Solution

FIG. 10

Primer

CGATATGGTTTGTTTCAAGGTATGATTTTTA

Blocker-I

AAGGTATGATTTTTAATTAAAAATTGTTGTTCACATTTGAAGG

......TAGCTATACCAAACAAAGTTCCATACTAAAAATTAATTTTT ‑‑‑ AACAAGTGTAAACTTCCA......

Target                                                                                NA18562

......TAGCTATACCAAACAAAGTTCCATACTAAAAATTAATTTTT AAC AACAAGTGTAAACTTCCA......

Variant                                          Detect Deletion                  NA18537

Target Cq = 26.3
ΔCq = 16.6

— NA18537
---- NA18562

FIG. 11A

Primer

CGATATGGTTTGTTTCAAGGTATGATTTTTA

Blocker-D
AAGGTATGATTTTTAATTAAAAA - - - TTGTTCACATTTGAAGG

......TAGCTATACCAAACAAAGTTCCATACTAAAAATTAATTTTT AAC AACAAGTGTAAACTTCCA......

Target                                                                  NA18537

......TAGCTATACCAAACAAAGTTCCATACTAAAAATTAATTTTT - - - AACAAGTGTAAACTTCCA......

Variant                                      Detect Insertion          NA18562

Target Cq = 27.8
ΔCq = 16.3

FIG. 11B

Primer  GTGGTAAATATTTACCTGGTCCCTG

Blocker
GGTAAAATAAACACCAAGACGTGGTAAATATTTAC

......GCCATTTTATTTGTGGTTCTCCACCATTTATAAATGGACCAGGGACA......
Target

......GCCATTTTATTTGTGGTTCTGCACCATTTATAAATGGACCAGGGACA......
Variant          BRAF rs3789806

FIG. 12

FIG. 13

Forward Primer

TGGAGCCTTGTATATAGACGGTAAAAT

Forward Blocker

GACGGTAAAATAAACACCAAGA C GTGGTAAA

...TGACCTCGGAACATATATCTGCCATTTTATTTGTGGTTCT C CACCATTTATAAATGGACCAGGGACAACAACTACAA...
Target
...TGACCTCGGAACATATATCTGCCATTTTATTTGTGGTTCT G CACCATTTATAAATGGACCAGGGACAACAACTACAA...
Variant

Reverse
Template

BRAF rs3789806

...ACTGGAGCCTTGTATATAGACGGTAAAATAAACACCAAGA C GTGGTAAATATTTACCTGGTCCCTGTTGTTGATGTT...
Variant
...ACTGGAGCCTTGTATATAGACGGTAAAATAAACACCAAGA G GTGGTAAATATTTACCTGGTCCCTGTTGTTGATGTT...
Target

Forward
Template

TTGTGGTTCT G CACCATTTATAAATGGACCA
Reverse Blocker

TATAAATGGACCAGGGACAACAACTAC
Reverse Primer

Target Cq = 32.2
ΔCq = 17.3

Target

Variant

RFU

FIG. 14

**EP 3 901 278 B1**

**REFERENCES CITED IN THE DESCRIPTION**

**Patent documents cited in the description**

- US 2014017685 A